# EUROPEAN PATENT APPLICATION

(11) **EP 2 258 712 A2**
(43) Date of publication of application: **08.12.2010**
(21) Application number: 10182423.3
(22) Date of filing: 14.03.2003
(51) Int. Cl.: C07H 21/04, A61K 48/00

(54) **Compositions and Methods to Initiate or Enhance Antibody and Major-histocompatibility Class I or Class II-restricted T Cell Responses by Using Immunomodulatory, Non-coding RNA Motifs**

(30) Priority: 15.03.2002 US 364490 P; 20.09.2002 US 412219 P
(62) Divisional of application: 03714172.8
(71) Applicant: Multicell Immunotherapeutics, Inc., San Diego, CA 92121 (US)
(72) Inventor: The designation of the inventor has not yet been filed
(74) Representative: Clegg, Richard Ian

(57) **Abstract**

The present application is directed to non-coding RNA motifs that are used in conjunction with an antigen or without an antigen to induce, enhance or modulate an immune response that comprises a B cell and a T cell component.

## Description

### Field of the Invention

The present invention relates generally to motifs that are useful in inducing an immune response. Specifically, the present application is directed to non-coding RNA motifs that are used in conjunction with an antigen or without an antigen to induce, enhance or modulate an immune response that comprises a B cell (antibody) and optionally a T cell component.

### Background of the Invention

A significant number of viral infections are associated with, or result in production of, RNA species that are not normally encountered in normal states. Such RNAs are either genomic fragments (in case of viruses containing double-stranded RNAs), replicative intermediates or stem-and-loop structures that are recognized by innate immune receptors such as TLR3 and trigger production of IFN-I and other soluble mediators. In addition, certain dsRNA motifs such as polyI:polyC (pI:pC or pI:C) have been shown to activate immature dendritic cells to a stage where they act as professional APC. Despite the fact that polyI:polyC and IFN-I have been shown to influence the antibody response to a protein antigen, most of the information obtained relative to dsRNA-immune modulatory motifs resulted from models of innate immunity, limited to natural killer cells, macrophages and other cell subsets devoid of specific antigen-receptors. Thus, it has not been demonstrated whether motifs associated with double-stranded or other RNA species have only a limited effect on the adaptive immune response, or act as potent danger signals that prevent immune tolerance and direct the differentiation of specific T cells. In addition, the critical question as to whether there is a multiplicity of RNA-associated danger motifs with potential differential impacts on immune response has not been addressed. Further, it has not been defined whether non-coding RNA motifs can facilitate the induction of class I-restricted immune responses during viral infections, thought until recently to occur, in the most part, subsequent to abortive or productive infection of antigen presenting cells (APC).

During viral infection, specific T lymphocytes are exposed to foreign epitopes displayed by MHC molecules and the B lymphocytes recognize antigens in soluble form. The proliferation and differentiation of lymphocytes define the adaptive immune response consisting of specific effector cells and memory cells. During the initial phase of the immune response, the innate immunity recognizes microbial associated motifs as well as lesion-triggered endogenous danger signals that direct the subsequent differentiation of specific lymphocytes and the overall profile of immune response. In the absence of danger signals, the T and B cell responses are reduced in magnitude and immune tolerance results, particularly at moderate to high doses of antigen. It has been proposed that this is a critical mechanism in discriminating between innocuous and 'dangerous' antigens associated with infection. This mechanism also sheds a different light on the strategy of immune system to discriminate between self and non-self, previously thought to be determined exclusively at the level of antigen-receptor repertoire.

### Summary of the Invention

The adaptive immune response is triggered by recognition of T and B cell epitopes and shaped by "danger" signals that act via innate immune receptors. In the present application it is shown that motifs associated with non-coding double stranded or single stranded RNA provide essential features to the immune response, reminiscent of viral infection, such as, rapid induction of pro-inflammatory chemokine expression, recruitment and activation of antigen presenting cells (APC), modulation of regulatory cytokines, differentiation of Th1 cells, isotype switching and stimulation of cross-priming, consisting in induction of MHC class I-restricted immune responses. The present application demonstrates the heterogeneity of RNA-associated motifs resulting in the differential impact on the profile of the immune response. Based on the ability of specific RNA-motifs to block tolerance induction and organize effectively the immune defense during viral infection, this demonstrates that such RNA species are potent "danger signals." The present application teaches using selected RNA motifs as adjuvants for optimizing the immune response to subunit vaccines. In conclusion, RNA-associated motifs that are produced during viral infection not have only a short-term impact on innate immunity, but bridge the early response with the late adaptive phase comprising activation and differentiation of antigen specific B and T cells.

In the present application, it is demonstrated that in addition to the single versus double stranded nature of RNA, the oligonucleotide composition is a critical determinant for recognition of non-coding RNA motifs by innate immune receptors. In addition, heterogenous RNA motifs have potent and differential impact on the adaptive immunity, mediating most of the features of the immune response during viral infection. Finally, it is also shown that newly described RNA-motifs effectively turn on defense mechanisms with prophylactic or therapeutic use in infectious diseases or cancers.

The present application claims priority to and incorporates by reference U.S. Serial No. 60/364,490 filed March 15, 2002 and U.S. Serial No. 60/412,219 filed September 20, 2002.

Various embodiments of the invention include:
1. A method of enhancing an immune response to an antigen comprising administering a composition comprised of double stranded RNA and coadministering said composition with said antigen.
2. A method of enhancing or modulating an immune response to an antigen, when the antigen is already present in the body, comprising administering a composition comprised of double stranded RNA.
3. The method of paragraph 1 wherein the RNA is non-coding RNA.
4. The method of paragraphs 1 or 2 wherein the double stranded RNA consists of poly-adenine and poly-uracil.
5. The method of paragraphs 1 or 2 wherein the double stranded RNA consists of one of the group consisting of poly-guanine and poly-cytosine or poly-inosine and poly-cytosine.
6. The method of paragraphs 1 or 2 wherein the double stranded RNA is comprised of adenine and uracil.
7. The method of paragraphs 1 or 2 wherein the double stranded RNA is comprised of guanine and cytosine or inosine and cytosine.
8. The method of paragraph 1 wherein the method enhances the Th1 and Tc1 cell response.
9. The method of paragraph 1 wherein the method induces Tc1 cell response.
10. The method of paragraph 1 wherein the method enhances the B cell response.
11. The method of paragraph 1 wherein said antigen is coadministered with an additional antigen.
12. The method of paragraph 1 wherein said method induces CXC and CC chemokines.
13. The method of paragraph 12 wherein said method induces MIP-1α, MIP-1β, MIP-3α and IP-10.
14. The method of paragraph 1 wherein said administering of said composition enhances T and B cell responses by recruiting and activating CD116+ monocytes or CD11c+ dendritic cells.
15. The method of paragraph 1 wherein double stranded RNA compositions enhance an immune response by recruiting antigen presenting cells.
16. The method of paragraph 1 wherein the antigen presenting cells are professional APC.
17. The method of paragraphs 1 or 2 wherein the double stranded RNA compositions comprise inosine and cytosine and induce effective recruitment of CD11c⁺ dendritic cells.
18. The method of paragraphs 1 or 2 wherein the double stranded is selected from the group consisting of an RNA composition comprising inosine and cytosine and an RNA composition comprising adenine and uracil and the method induces effective recruitment of CD11b+ monocytes.
19. The method of paragraph 15 wherein the APC is activated.
20. A method of paragraph 4 wherein the antigen is a noninfectious antigen and the RNA MHC class I-restricted T cells are cross-primed by the poly-adenine and poly-uracil RNA composition.
21. The method of paragraphs 4 - 7 wherein the RNA composition is administered mucosally.
22. A method of preventing high-zone tolerance to a non-infectious antigen comprising administering said non-infectious antigen together with a double stranded RNA composition comprising either poly-adenine and poly-uracil or poly-inosine and poly-cytosine.
23. The method of paragraph 22 wherein the non-infectious antigen is administered in high doses or is already present in the body.
24. The method of paragraph 22 wherein the dose is a toleragenic amount of antigen.
25. The method of paragraph 22 wherein the method prevents B cell unresponsiveness.
26. The method of paragraphs 1, 2 and 22 wherein the composition and antigens are administered by one of the following: mucosal administration; respiratory administration; intravenous administration; subcutaneous administration; and intramuscular administration, complexed or not to various carriers.
27. A method of immunization comprised of loading an antigen presenting cell by use of at least one peptide epitope of an antigen attached to an Ig backbone thereby forming an Ig -peptide molecule and administering *in vivo* the Ig-peptide molecule in conjunction with a dsRNA motif wherein the epitope is effectively processed and presented by the MHC I pathway resulting in effective loading of MHC class I molecules and thereby resulting in an effective secondary expansion of MHC class I-restricted T cells subsequent to *in vivo* exposure to the antigen.
28. The method of paragraph 27 wherein the antigen is a virus.
29. The method of paragraph 28 wherein the virus is the influenza virus.
30. The method of paragraph 27 wherein the peptide-epitope is recIgG-NP(Kd).
31. The method of paragraph 27 wherein the dsRNA is pA:pU.
32. The method of paragraph 27 wherein the T cells are cytotoxic T lymphocytes.
33. The method of paragraph 27 wherein the secondary expansion of MHC class I-restricted T cells subsequent to *in vivo* exposure to the antigen is greater than administration of the recombinant antigen in sterile saline only.
34. A method of controlling and treatment of a tumor after clinical diagnosis, by loading an antigen presenting cell by use of at least one tumor associated T cell epitope attached to an IgG backbone thereby forming an IgG -peptide molecule and administering the Ig-peptide molecule *in vivo* in conjunction with dsRNA.
35. The method of paragraph 34 wherein the tumor associated T cell epitope is effectively processed and presented by the MHC I pathway resulting in effective loading of MHC class I molecules thereby resulting in an MHC class I - peptide complex.
36. The method of paragraph 34 wherein the method results in an immune response to the tumor associated T cell epitope and tumor rejection.
37. The method of paragraph 34 wherein the dsRNA is pA:pU.
38. The method of paragraph 34 wherein the Ig-G peptide complex and dsRNA are administered repeatedly as an anti-tumor therapy.
39. The method of paragraphs 34 and 35 wherein upon tumor rejection Tc1 immunity is developed against the tumor associated epitope.
40. The method of paragraph 34 where upon administration of IgG-peptide and dsRNA, Tc2 immunity is developed against the tumor associated epitope.
41. The method of paragraph 34 wherein the method further induces an effective memory response to the same tumor associated epitope.
42. The method of paragraph 34 wherein the method results in continued immunity to tumor cell variants.
43. A method of enhancing an antibody response to an antigen comprising administering said antigen to a human or nonhuman mammal in conjunction with dsRNA selected from the group consisting of pA:pU, pI:pC and pC:pG or mixtures thereof.
44. A method of enhancing an antibody response to an antigen comprising administering said antigen to a human or nonhuman mammal in conjunction with mixtures of single stranded RNA species selected from the group consisting of pA, pC, pI, pU, p(G,U), p(C,U), p(A,C), p(I,U), p(C,I), p(A,U), p(A,G), p(A,C,G), p(A,C,U) and p(A,G,U).
45. A composition for enhancing an immune response to an antigen comprising a dsRNA sequence consisting of poly-adenine and poly-uracil.
46. The composition of paragraph 45 wherein the composition further comprises the antigen.
47. The composition of paragraph 45 wherein the antigen is already present in the body.
48. The composition of paragraph 45 wherein said antigen is administered in a pharmaceutically acceptable carrier.
49. The composition of paragraph 45 wherein said antigen is administered in an immunoglobulin.
50. The composition of paragraph 48 wherein said pharmaceutically acceptable is IgG.
51. The composition of paragraphs 45 - 47 wherein the antigen is a tumor associated epitope.
52. The composition of paragraphs 45 - 47 wherein the antigen is a virus.
53. The composition of paragraph 51 wherein the antigen is a tumor associated T cell epitope.
54. The composition of paragraphs 45 - 53 wherein the dsRNA is administered together with said antigen.
55. The composition of paragraphs 45 - 53 wherein said dsRNA is administered separately from said antigen.
56. A composition for enhancing an immune response to an antigen comprising a dsRNA sequence consisting of poly-inosine and poly-cysteine.
57. The composition of paragraph 56 wherein the composition further comprises the antigen.
58. The composition of paragraph 56 wherein the antigen is already present in the body.
59. The composition of paragraph 56 wherein said antigen is administered in a pharmaceutically acceptable carrier.
60. The composition of paragraph 56 wherein said antigen is administered in an immunoglobulin.
61. The composition of paragraph 59 wherein said pharmaceutically acceptable is IgG.
62. The composition of paragraphs 56 - 58 wherein the antigen is a tumor associated epitope.
63. The composition of paragraphs 56 - 58 wherein the antigen is a virus.
64. The composition of paragraph 62 wherein the antigen is a tumor associated T cell epitope.
65. The composition of paragraphs 56 - 64 wherein the dsRNA is administered together with said antigen.
66. The composition of paragraphs 56 - 64 wherein said dsRNA is administered separately from said antigen.
67. Use of double stranded ("dsRNA") for the manufacture of a medicament for enhancing an immune response to an antigen, comprising:
   administering said dsRNA to a patient in conjunction with said antigen.
68. Use of dsRNA for the manufacture of medicament for enhancing an immune response to an antigen, comprising:
   administering said dsRNA to a patient when the antigen is already present in the body of the patient.
69. The use of paragraphs 67 or 68 wherein the dsRNA is non-coding RNA.
70. The use of paragraphs 67 or 68 wherein the double stranded RNA consists of poly-adenine and poly-uracil.
71. The use of paragraphs 67 or 68 wherein the double stranded RNA consists of poly-inosine and poly-cytosine or poly-guanine and poly-cytosine.
72. The use of paragraphs 67 or 68 wherein the double stranded RNA is comprised of adenine and uracil.
73. The use of paragraphs 67 or 68 wherein the double stranded RNA is comprised of guanine and cytosine or inosine and cytosine.
74. The use of paragraphs 67 - 73 wherein the use enhances the Th1 and Tc1 cell response.
75. The use of paragraphs 67 - 73 wherein the use induces Tc1 cell response to the antigen.
76. The use of paragraphs 67 - 73 wherein the method enhances the B cell response to the antigen.
77. The use of paragraphs 67 or 68 wherein said antigen is coadministered with an additional antigen.
78. The use of paragraphs 67 or 68 wherein said method induces CXC and CC chemokines.
79. The use of paragraphs 67 or 68 wherein said method induces MIP-1α, MIP-1β, MIP-3α and IP-10.
80. The use of paragraphs 67 - 71 wherein said administering of said dsRNA enhances T and B cell responses by recruiting and activating CD11b+ monocytes or CD11c+ dendritic cells.
81. The use of paragraphs 67 - 71 wherein double stranded RNA compositions enhance an immune response by recruiting antigen presenting cells.
82. The use of paragraph 81 wherein the antigen presenting cells are professional APC.
83. The use of paragraph 71 wherein the double stranded RNA compositions comprise inosine and cytosine and induce effective recruitment of CD11c⁺ dendritic cells.
84. The use of paragraphs 67 or 68 wherein the double stranded is selected from the group consisting of an RNA composition comprising inosine and cytosine and an RNA composition comprising adenine and uracil and the method induces effective recruitment of CD11b+ monocytes.
85. A use of paragraphs 67 - 70 wherein the antigen is a noninfectious antigen and the RNA MHC class I-restricted T cells are cross-primed by the poly-adenine and poly-uracil RNA composition.
86. The use of paragraphs 67 - 70 wherein the composition and antigens are administered by one of the following: mucosal administration; respiratory administration; intravenous administration; subcutaneous administration; and intramuscular administration, complexed or not to various carriers.
87. Use of dsRNA for the manufacture of medicament for preventing high-zone tolerance to a non-infectious antigen comprising administering said non-infectious antigen together with a double stranded RNA composition comprising either poly-adenine and poly-uracil or poly-inosine and poly-cytosine.
88. The use of paragraph 87 wherein the non-infectious antigen is administered in high doses or already present in the body.
89. The use of paragraph 87 wherein the dose is a toleragenic amount of antigen.
90. The use of paragraph 87 wherein the method prevents B cell unresponsiveness.
91. The use of dsRNA for the manufacture of a medicament comprising loading an antigen presenting cell by use of at least one peptide epitope of an antigen attached to an Ig backbone thereby forming an Ig -peptide molecule and administering in vivo the Ig-peptide molecule in conjunction with a dsRNA motif wherein the epitope is effectively processed and presented by the MHC I pathway resulting in effective loading of MHC class I molecules and thereby resulting in an effective secondary expansion of MHC class I-restricted T cells subsequent to in vivo exposure to the antigen.
92. The use of paragraph 91 wherein the antigen is a virus.
93. The use of paragraph 91 wherein the virus is the influenza virus.
94. The use of paragraph 91 wherein the peptide-epitope is recIgG-NP(Kd).
95. The use of paragraph 91 wherein the dsRNA is pA:pU.
96. The use of paragraph 91 wherein the T cells are cytotoxic T lymphocytes.
97. The use of dsRNA in the manufacture of a medicament for controlling and treatment of a tumor after clinical diagnosis, comprising loading an antigen presenting cell by use of at least one tumor associated T cell epitope attached to an IgG backbone thereby forming an IgG -peptide molecule and administering the Ig-peptide molecule *in vivo* in conjunction with a dsRNA.
98. The use of paragraph 97 wherein the tumor associated T cell epitope is effectively processed and presented by the MHC I pathway resulting in effective loading of MHC class I molecules thereby resulting in an MHC class I - peptide complex.
99. The use of paragraph 97 wherein the method results in an immune response to the tumor associated T cell epitope and tumor rejection.
100. The use of paragraph 97 wherein the dsRNA is pA:pU.
101. The use of paragraph 97 wherein the Ig-G peptide complex and dsRNA are administered repeatedly as an anti-tumor therapy.
102. The use of paragraph 97 wherein upon tumor rejection Tc1 immunity is developed against the tumor associated epitope.
103. The use of paragraph 97 where upon administration of IgG-peptide and dsRNA, Tc2 immunity is developed against the tumor associated epitope.
104. The use of paragraph 97 wherein the method further induces an effective memory response to the same tumor associated epitope.
105. The use of paragraph 97 wherein the method results in continued immunity to tumor cell variants.
106. The use of dsRNA in the manufacture of a medicament for enhancing an antibody response to an antigen comprising administering said antigen to a human or nonhuman mammal in conjunction with dsRNA selected from the group consisting of pA:pU, pI:pC and pC:pG.
107. The use of dsRNA in the manufacture of a medicament for enhancing an antibody response to an antigen comprising administering said antigen to a human or nonhuman mammal in conjunction with mixtures of single stranded RNA species selected from the group consisting of pA, pC, pI, pU, p(G,U), p(C,U), p(A,C), p(I,U), p(C,I), p(A,U), p(A,G), p(A,C,G), p(A,C,U) and p(A,G,U).
108. The use of dsRNA in the manufacture of a medicament for enhancing an immune response to an antigen comprising a dsRNA sequence consisting of poly-adenine and poly-uracil.
109. The use of paragraph 108 wherein the composition further comprises an antigen.
110. The use of paragraph 108 wherein the antigen is already present in the body.
111. The use of paragraph 108 wherein said antigen is administered in a pharmaceutically acceptable carrier.

### Brief Description of the Drawings:

Figure 1 shows the effect of various synthetic RNA motifs on the specific antibody and T cell immunity;
Figure 2 demonstrates the increase of immune response to viral antigens by a specific dsRNA motif;
Figure 3 shows the impact of defined dsRNA motifs on innate immunity and antigen presenting cells;
Figure 4 illustrates the "danger-signal" quality of specific dsRNA motifs;
Figure 5 demonstrates the use of selected dsRNA motifs as potent vaccine adjuvants;

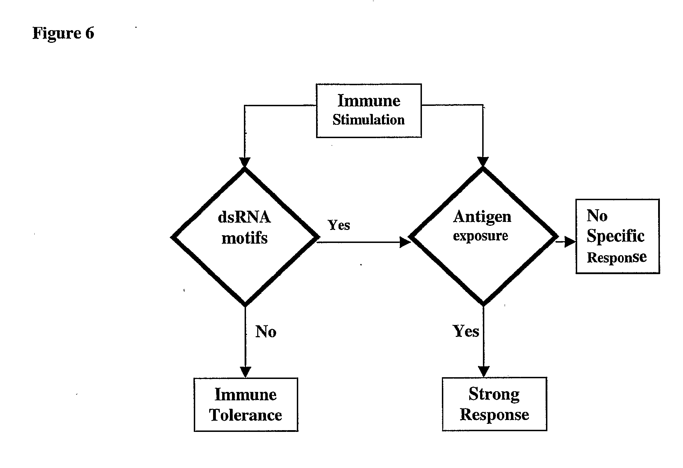

Fig. 6 is a flow chart demonstrating the effect of dsRNA motifs on an immune response.
Fig. 7 shows that shows that natural, non-infectious double stranded RNA produced during infection with influenza virus, has substantial effects on the specific immune response to a protein antigen;
Fig. 8A shows an extensive library of synthetic RNA motifs;
Fig. 8B shows that different synthetic RNAs have an enhancing effect on the B and T cell response to a prototype protein antigen;
Fig. 9 shows effects of selected RNA motifs on the innate immune response;
Fig. 10 shows that distinct RNA motifs bind to different receptors on antigen presenting cells;
Fig. 11 shows that distinct RNA motifs induce differential upregulation of chemokines;
Fig. 12 shows that the control of replication of influenza virus can be achieved by using selected synthetic RNA motifs;
Fig. 13 shows that selected synthetic RNA motifs pI:pC and pA:pU largely prevent high zone tolerance that is usually associated with administration of large amounts of purified protein;
Fig. 14 shows that selected synthetic RNA motifs effect on human monocytic cells;
Figs. 15A - 15B show that non-tagged pA:pU, but not non-tagged pI:pC, was able to compete out the binding of tagged pA:pU to human THP-1 monocytic cells;
Fig. 16 shows the purification and fractionation steps of dsRNA;
Fig. 17 shows that lower molecular weight fractions of a selected synthetic RNA compounds are endowed with higher biological activity;
Fig. 18 shows that pI:pC but not pA:pU induced antibody response against itself, with a cross-reactive component against another RNA motif;
Fig. 19 shows that co-use of selected synthetic RNAs promote effective induction of IL-2 and IFN-gamma subsequent to IgG mediated delivery of an MHC class I-restricted epitope;
Fig 20 shows that *ex vivo* APC loading by recombinant IgG is more effective in formation of MHC class I-peptide complexes and generation of Tc response, compared to use of the peptide itself;
Fig. 21 shows that IgG mediated delivery of a class I restricted epitope is most effective in priming class I restricted Tc1 responses when co-administration of selected synthetic RNA was carried out;
Fig. 22 shows that effective priming of anti-viral cytotoxic T cells requires both effective *in vivo* loading of APC with class I restricted epitope delivered via IgG, together with appropriate instruction by selected synthetic RNA motif;
Fig. 23 shows that immunization with a recombinant IgG bearing a viral class I restricted epitope together with selected synthetic dsRNA, resulted in priming of an immune response capable to limit the replication of a virus subsequent to infectious challenge;
Fig. 24 describes the tumor models used for testing the efficiency of a Ig-peptide-based molecules;
Fig. 25 shows that both effective *in vivo* loading of APC with tumor associated antigen, together with simultaneous activation by selected synthetic RNA motifs, are necessary and sufficient for effective control of tumor growth and induction of tumor rejection;
Fig. 26 shows that both effective *in vivo* loading of APC with tumor associated antigen, together with simultaneous activation by selected synthetic RNA, can trigger an effective immune response to tumor-associated antigens;
Fig. 27 shows that tumor infiltrating lymphocytes displaying the T cell receptor marker TCRβ acquired expression of the activation marker CD25 upon treatment with recombinant immunoglobulin bearing tumor associated epitope, together with selected synthetic dsRNA motif;
Fig. 28 shows that the treated mice that successfully rejected the tumor developed Tc1 responses against the tumor associated epitope on the therapeutic Ig, along with Tc2 immunity;
Fig. 29 shows that successful rejection of tumor induced by indicated treatment is followed by effective protection against subsequent challenge with the same tumor, indicating development of effective immune memory;
Figs. 30A - 30B show that the emerging immunity, subsequent to the indicated treatment that results in tumor rejection, protects against challenge with loss of antigen variants and is associated with overall expansion of cytokine producing cells;
Fig. 31A shows: (a) representation of natural IgG (light chain - heavy chain heterodimer); (B) antigen (Ag) derived peptide inserted within CDR (complementarity determining region) 3, 2, 1 or framework region; (C) VH segment replaced with an antigen or fragment; and, (D) VH and CH1 segments replaced with antigen or antigen fragment;
Fig. 31B diagramatically illustrates the IgG-peptide and Fc peptide;
Fig. 31C shows properties of selected human IgG backbone; and,
Fig. 31D shows the sequence of the constant region of the heavy chain as well as schematic depiction of a prospective construct.

### Detailed Description of the Invention

The mechanisms of immune response during microbial infection became a major area of investigation subsequent to advances in understanding the potential role of innate immunity. It quickly became evident that the innate immune cells are endowed with multiple categories of receptors that discriminate between various microbial-associated motifs or "exogenous" danger signals. Communication between innate and adaptive immunity subsequent to such pattern recognition events decisively influences the magnitude and profile of T and B cell responses. The innate immunity is rapid though less discriminative, but instructs the adaptive immunity that evolves slower and is composed of more potent effectors, with a vast repertoire acquired by somatic mutation. This multi-patterned recognition strategy that employs jointly the innate and adaptive immunity shifted the immune discrimination paradigm from self/non-self to dangerous/non-dangerous cognition. The poor immunogenicity of purified proteins, induction of immune mediators by microbial motifs and characterization of the activity of such mediators (cytokines, chemokines and co-stimulatory molecules) on adaptive immunity, all supported this concept.

As described in the present application, a rational approach in delineating the role of non-coding RNA motifs as danger signals was taken, with direct implications for understanding their role in controlling the adaptive immunity during viral infections. In addition, their use as adjuvants in conjunction with vaccination is explored.

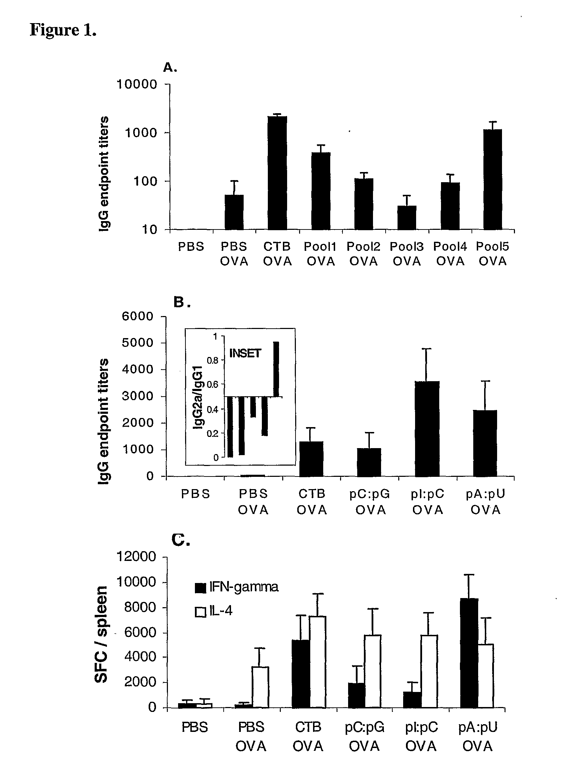

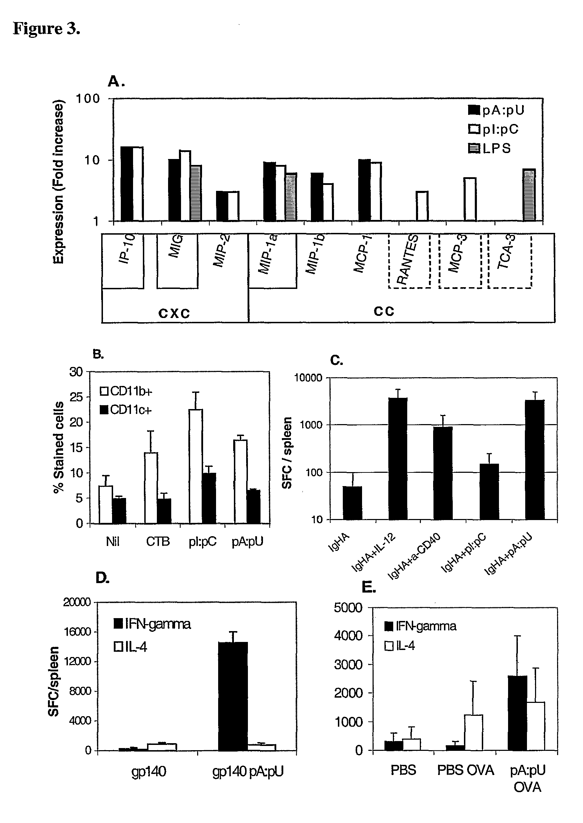

A library of synthetic RNAs and a two-tier strategy was employed, using as readout the effect on the adaptive rather than innate immunity. By this method, it was surprisingly found that the oligonucleotide composition in addition to the double stranded nature of RNA plays a role in this concern. It is shown that A:U-based motifs have the ability to turn on the Th1 immunity, isotype switching to IgG2a (Figs. 1A - 1C) and cross-priming (Fig. 3A - 3E) to a higher extent than the I:C-based motifs. Such I:C motifs, defined earlier, result in enhanced T2 and B cell immunity (Figs. 1A - 1C). C:G-motifs associated with dsRNA, or miscellaneous ssRNA motifs, displayed low effect on adaptive immunity, unless mixtures of ssRNAs composed of complementary bases were used. Since these findings have been reproduced in the absence of functional TLR4, a pathway common with that of endotoxin recognition can be ruled out at this point. Recently, it has been shown that TLR3 plays a role in recognition of pI:pC but a common recognition pathway with pA:pU seems unlikely due to the profoundly different profile of the immune response triggered by these two motifs (Figs. 1A - 1C). More likely, different TLR isoforms and/or co-receptors participate to the process of discriminating RNA motifs based on identity of nucleotides, in a process reminiscent of a rudimentary immune repertoire. A possibility may be that TLR9 shown to recognize palindromic unmethylated CpG oligodeoxynucleotide motifs or isoforms of TLR, may be involved in dsRNA-motif discrimination. Recent evidence does not support the involvement of TLR9 since dsRNA induces a different spectrum of transcription factors and co-stimulatory molecules as compared to unmethylated CpG motifs. Since both pI:pC and pA:pU induce CXC chemokines (Figs. 3A), it is likely that alternative mediators such as CC chemokines with ability to bind selectively Th2 cells, may be responsible for the different Th profile elicited by these motifs.

Based on the data, it is concluded that the newly characterized pA:pU-associated motifs can induce a large number of features of the adaptive immune response, that are usually noted only subsequent to viral infection. Induction of T1 responses (both Th1 and Tc1) was documented with protein antigens (OVA and gp140) and inactivated influenza virus (Figs. 1-3). Induction of MHC class I-restricted response to protein antigens (Fig. 3 D,E) suggests that this RNA motif was sufficient to activate APC to a level compatible with this mechanism of processing and presentation, adding novel information that supports cross-priming as major mechanism in viral infections. This suggests that RNA-associated danger motifs rather than direct infection of APC may be responsible for induction of cytotoxic T lymphocytes (CTL) during infection with RNA viruses, such as influenza virus. The enhanced magnitude of the immune response can be explained by rapid recruitment and activation (Figs. 3A - 3E) of APC. The induction of T1 immunity promoted by pA:pU is accompanied by isotype switching, resulting in generation of IgG2a antibodies (Fig. 1B). However, dsRNA could not induce isotype switch to the IgA class. This was associated with inhibition of TGF-β (not shown), suggesting that dsRNA danger motifs act by virtue of induction of pro-inflammatory and down-regulation of anti-inflammatory mediators. These results are consistent with a considerable, but not exclusive, role of dsRNA motifs in shaping up the adaptive immunity during infections such as influenza.

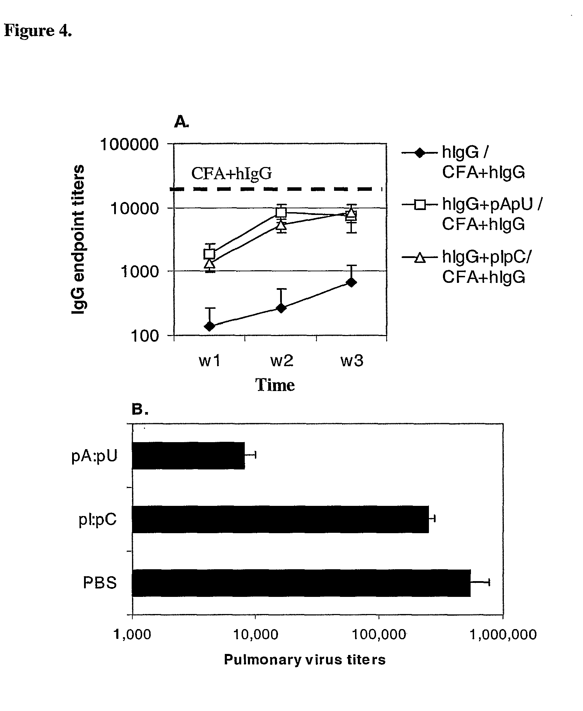

Since potent danger motifs should influence the outcome in terms of immune responsiveness versus tolerance, it was studied whether dsRNAs prevent high-zone tolerance to human IgG, a well-characterized model of immune unresponsiveness. It is shown that both pA:pU and pI:pC are potent inhibitors of immune tolerance (Figs. 4A - 4B), not only modulators of the adaptive response. This observation enriches the panel of features born by dsRNAs and opens up the possibility that adjuvant effects of danger motifs in general, are at least in part caused by prevention of immune unresponsiveness. Lastly, since A and U, but not I (inosine) bases, are found in natural RNA species, the data point to the first dsRNA motif with potential relevance to the immune response during viral infections.

The potency of pA:pU as a danger motif is illustrated by its ability to control primary infection with influenza virus (Fig. 4A - 4B). This feature that can be explained by rapid mobilization of innate and adaptive responses, is highly reminiscent of the ability of unmethylated CpG oligoDNA motifs to improve on immune defense during primary infection. By extrapolation, it is concluded that the innate immunity has an exquisite ability to recognize foreign or endogenously generated, infection-associated polynucleotide motifs and regulate accordingly the adaptive immunity. Thus, when antigen is present in the system, additional exposure to dsRNA may simply mobilize more effectively the immune responses, with direct implication on clearance of the antigen.

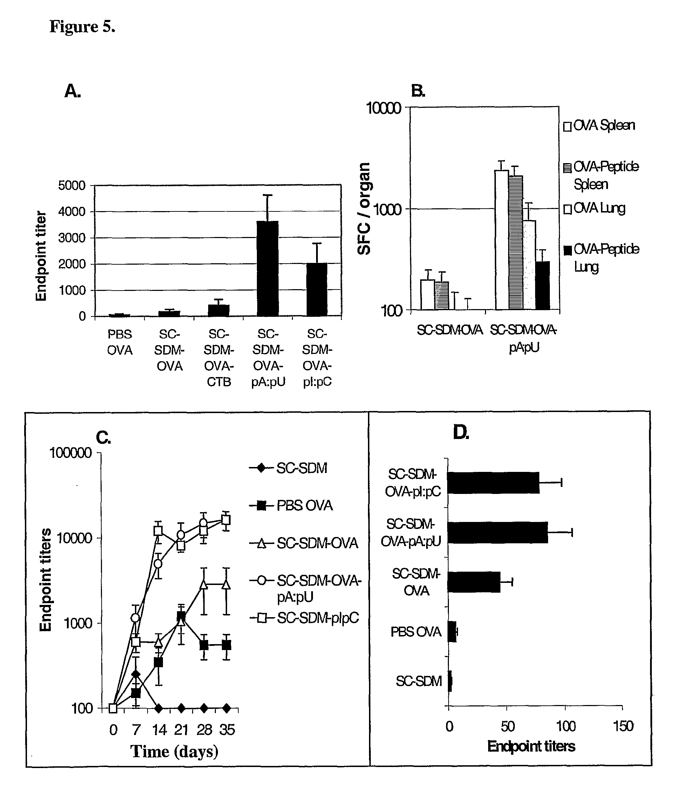

Based on the results stated herein, dsRNA motifs are logical candidates for adjuvants in conjunction with subunit, recombinant or inactivated vaccines. In particular, pA:pU appears likely to provide some of the beneficial features of live vaccines in the absence of vector replication. The present application describes immunological complexes for mucosal and systemic vaccination that allowed co-formulation of antigen and dsRNA. As shown in the Figs. 5A - 5D, pulmonary vaccination and cancer immunotherapy with such complexes result in induction of a robust immune response, consisting of antibodies, T helper and class I-restricted T cells.

In conclusion, by using a rational approach of selecting RNA motifs that impact the adaptive immune responses, an unexpected heterogeneity and novel RNA-associated danger motifs were defined. A systematic study of the adaptive immune response demonstrated that selected RNA motifs orchestrate a broad range of features that are reminiscent of natural infection. Finally, the present application study defines novel formulations containing such RNA motifs that are of potential use in mucosal or systemic vaccination, as well as immunotherapy by eliminating immune unresponsiveness or tolerance.

### A) Materials and Methods

### 1) Antigens and immunomodulator

A panel of 18 single-stranded and double stranded synthetic RNAs (see Table 1) was purchased from Sigma and dissolved in sterile PBS. The RNAs were used as pools or individually. Ovalbumin (OVA, low endotoxin) was purchased from Sigma (A7641). Cholera toxin subunit B (CTB) from Calbiochem (catalog # 227039), Complete Freund's adjuvant (CFA) from DIFCO (catalog #263810) and human IgG (hIgG) from Sigma (catalog # I 4506). The recombinant gp140 HIV antigen that retains conformational epitopes and has the ability to trimerize, was derived from gp160 envelope protein of the strain IIIB by introducing a stop mutation. The antigen was expressed by a vaccinia virus vector generously provided by Dr. Bernard Moss (N.I.H.), in BS-C-1 (ATCC) cells and purified by lentil lectin sepharose chromatography (Pharmacia, Piscataway, NJ). The identity of gp140 antigen was confirmed by Western blot analysis using HIV envelope-specific antibodies purchased from Fitzgerald (Catalog # 20-HG81). Influenza virus (strain A/WSN/32 H1N1) was grown on MDBK cells and purified from supernatants by sucrose-gradient centrifugation. For virus-inactivation, the virions were exposed to shortwave UV light for 15 minutes under stirring. The inactivation was confirmed by virus titration on permissive MDCK cells. Recombinant mouse IgG₂b bearing the I-E^{d}-restricted hemagglutinin-derived peptide SFERFEIFPKE (IgHA) [Seq. I.D. No. 1] within the variable region was obtained and purified as characterized previously.

**Table 1**

| **Category** | | **Composition** |
|---|---|---|
| **Single-stranded RNA** | Group 1 (Pool 1) | p*(A); p(C); p(G); p(I); p(U) |
| | Group 2 (Pool 2) | p(G,U); p(C,U); p(A,C); p(I,U) |
| | Group 3 (Pool 3) | p(C,I); p(A,U); p(A,G) |
| | Group 4 (Pool 4) | p(A,C,G); p(A,C,U); p(A,G,U) |
| **Double-stranded RNA** | Group 5 (Pool 5) | pC :pG; pA:pU; pI:pC |

| | | |
|---|---|---|
| * p="poly" | | |

### 2) Animals

C57BL/6, BALB/c and TLR4-/- C3H/HeJ female mice, between 6-8 weeks of age, were purchased from the Jackson Laboratories (Bar Harbor, MA) and housed under specific pathogen conditions at Alliance Pharmaceutical Corp. Key observations in C57BL/6 and BALB/c mice were reproduced in C3H/HeJ mice that have deficient responsiveness to endotoxin. Female Sprague Dawley rats (250-330 grams) were purchased from Taconic farms and housed in similar conditions.

### 3) Immunization, challenge and measurement of virus titers

Mice and rats were primed by intratracheal instillation or aerosolization respectively, as described before and in the case of mice, boosted twice intranasally, at 2 weeks interval. For induction of high-zone tolerance, the mice were primed by intravenous injection. Finally, for the induction of strong immune responses, mice were immunized subcutaneously with antigen emulsified in CFA. The amounts of antigens used for priming, boosts or induction of tolerance were: OVA - 100µg; HIV gp140 - 10µg; hIgG - 200µg; and sucrose-purified UV-WSN - 20µg. The amount of synthetic RNA used was 40-50µg/dose with or without antigen, incorporated or not in SCL COMPLEXES. The amount of CTB / dose was of 10µg. The antigens were either delivered in saline or when formulated, in perfluorocarbon (perflubron [neat perfluorooctyl bromide], Liquivent®, Alliance Pharmaceutical Corp.) which is an inert vehicle that is compatible with the SCL matrix (total volume of instillation or aerosolization, of 40-45µl).

For virus challenge, C57BL/6 and TLR4-/- C₃H/HeJ mice under Metofane anesthesia were infected with sublethal doses (10⁴ tissue culture infective doses 50% - TCID₅₀) of live WSN virus, via the nasal route. On day 5 after infection, the mice were sacrificed, lungs retrieved, homogenized and stored at -70°C. The virus titers were measured by 48-hour incubation of serial dilutions of samples with permissive MDCK cells, followed by standard hemagglutination with chicken red blood cells (From Animal Technologies). The endpoint titers were estimated in triplicate measurements by interpolation and expressed as TCID₅₀ / organ.

### 4) Immunological complexes

The technological process of obtaining short chain lipid complexes ("immunological complexes") with phospholipid as major excipient was spray drying. A more simple version of this process was used herein. In brief, the phospholipid was homogenized in water (to form liposomes or micelles) and mixed with the excipients and the actives, followed by spray drying, as described in detail: an aqueous preparation was prepared by mixing two preparations, A and B, immediately prior to spray-drying. Preparation A was comprised of a micellar preparation in which 0.14 g of dioctanoylphosphatidylcholine (Avanti Polar Lipids) by dissolving the phospholipid in 23 mL of hot DI water. 0.0357 g of CaCl₂•2H₂O and 0.714gr of Lactose was dissolved in the phospholipid micellar preparation. Preparation B was comprised of 20 mg of Ovalbumin (Sigma) and 4 mg of pA:pU (endotoxin free) which was dissolved in 5 mL of PBS. The combined feed preparation ( 2 mL of preparation A with preparation B) and was spray dried with a standard B-191 Mini spray drier under the following conditions: inlet temperature = 70°C, outlet temperature = 43°C, aspirator = 90%, pump = 2.2 mL/min, nitrogen flow = 2400 L/h. The resulting complex had a PL: OVA: pApU: CaCl₂•2H₂O: Lactose weight ratio of 12:20: 4: :3:61.

### 5) Measurement of antibody and T cell response

The antibody response was measured by ELISA. In brief, wells were coated with antigen (2µg/ml of gp140, 8µg/ml of sucrose purified virus, 10µg/ml of hIgG or OVA, respectively) and blocked with SeaBlock (Pierce, Rockford, IL, catalog # 37527). Serial dilutions of serum and bronchoalveolar lavage fluid were incubated for at least 2 hours at room temperature. After washing, the assay was developed with anti-mouse IgG antibody coupled with alkaline phosphatase (Sigma, cat# A7434) followed by addition of substrate (pNPP, Sigma, cat# N2765) and measurement by using an automatic ELISA reader (Molecular Devices, ThermoMax) equipped with SoftMax software.

For the measurement of cellular response, splenic cell suspensions were obtained by passing the organ through 70 micron nylon Falcon strainers (Becton Dickinson, cat# 352350) followed by lysis of red blood cells with red blood cell lysis buffer (Sigma, cat# R7757). The lymphocytes from the pulmonary associated lymphoid tissue were isolated by collagenase (Sigma, cat# C9891) digestion of lung tissue followed by Ficoll-Paque (Amersham Pharmacia, cat# 17-1440-02) gradient centrifugation. The T cell response was measured by ELISPOT analysis: 96-well 45 micron mixed cellulose ester plates (Millipore, cat#MAHA S4510) were coated with 4µg/ml of rat anti-mouse anti-IFNγ, IL-2 or IL-4 monoclonal antibodies (BD-PharMingen, cat#554430, cat#18161D, cat# 554387 respectively). After blocking with 10% FCS in sterile saline for 1 hour at 37°C, spleen cell suspensions were added at 5x10⁵ cells / well together or without antigens or peptides. In the case of pulmonary lymphocytes, effector cells were 1:1 mixed with mitomycin-treated, splenic stimulator cells before stimulation. For stimulation, graded amounts of antigen were used (OVA, gp140, hIgG or sucrose-purified WSN virus) or peptides: class II-restricted HA SFERFEIFPKE [Seq. I.D. No. 1]; or class I-restricted SIINFEKL [Seq. I.D. No. 2] and HIV V3-derived R10K peptide described previously. At 72 hours after stimulation, the assay was developed with biotinylated rat anti-mouse cytokine antibodies (BD-PharMingen) followed by streptavidin-HRP (BioSource Int., Camarillo, CA) and insoluble ABC substrate. The results were measured using an automatic imaging system (Navitar/Micromate) equipped with multiparametric-analysis software (Image Pro, Media Cybernetics).

### 6) Measurement of chemokine gene-expression

The level of chemokine expression in the lungs of mice treated 1 day previously with synthetic RNA or controls was measured by DNA array technology as follows: total RNA was isolated from lungs using an RNeasy kit (Qiagen, Valencia, CA). The RNAs were further purified by treatment with RNase-free DNase I (Stratagene, San Diego, CA). DNA array was performed by using the Nonrad-GEArray kit from SuperArray Inc. (Bethesda, MD). Briefly, cDNA probes were synthesized using MMLV reverse transcriptase with dNTP mix containing biotin-16-dUTP. The GEArray membranes were prehybridized at 68°C for 1-2 hours. The hybridization was carried out by incubation of the membranes with biotin-labeled cDNA. The hybridized membranes were washed in 2xSSC - 1% SDS twice and 0.1xSSC - 0.5% SDS twice. The membranes were further incubated with alkaline phosphatase-conjugated streptavidin (BioSource Int., Camarillo, CA) and finally developed with CDP-Star chemiluminescent substrate. The intensity of signal was measured with Image-Pro analysis system equipped with Gel-Pro software (Media Cybernetics, Silver Springs, MD).

### 7) Flow Cytometry

Cell suspensions from lungs of mice, treated one day previously with synthetic RNA or saline, were prepared by collagenase digestion and Ficoll gradient centrifugation, as above. The cells were resuspended in phosphate buffered saline containing 1% (v:v) mouse serum (Sigma. cat# M5905) and 1% (w:v) bovine albumin, fraction V (Sigma, cat# A3059) and stained with PE-labeled rat anti-mouse CD11b (PharMingen, cat# 01715A), PE-labeled hamster anti-mouse CD11c (PharMingen, cat# 09705A) or the appropriate PE-labeled isotype control (PharMingen, cat# 11125A or 11185A) at a concentration of 1µg antibody / 10⁶ cells, for 40 minutes on ice. The analysis was carried out on a Becton Dickinson FacsCalibur instrument. The non-viable cells were gated out using propidium iodide.

### 8) Magnetic separation and adoptive transfer

Professional APC such as CD11c⁺ dendritic cells were separated from spleens of BALB/c mice by using magnetic beads coupled to rat anti-mouse anti-CD11c antibodies (Miltenyi Biotech). In brief, single cells suspensions were resuspended in MACS buffer ( BSA and EDTA) at 10⁷ cells/ ml, incubated for 15' on ice with magnetic beads, washed and passed through magnetic columns. The columns were washed three times before elution, followed by two consecutive washings and *in vitro* overnight pulsing with 100µg/ml of IgHA together or without 50µg/ml RNA motifs, or 5ng/ml rIL-12 (Biosource Int., Camarillo, CA). Alternatively, the cells were incubated overnight with IgHA on wells previously coated with rat anti-mouse CD40 monoclonal antibody (BD-PharMingen). The cells were washed, resuspended in balanced sterile saline and adoptively transferred by subcutaneous injection into naïve BALB/c mice (2.5x10⁵ APC / mouse). The T cell response was measured at 14 days by IL-2 ELISPOT analysis subsequent to stimulation with HA class II-restricted peptide, as described above.

### 9) Statistical analysis

Magnitudes of immune responses were compared by using the t test, assuming a normal distribution of the values and equal variances.

### B. Results

### 1) Systematic definition of RNA motifs that modulate immune responses

During viral infection, transient, unusual RNA species are created and act as "danger" signals. It was therefore postulated that multiple, various RNA motifs are being recognized by the innate immune cells and profoundly regulate the adaptive immune response. To address this hypothesis on a systematic basis, a library of synthetic, single-stranded and double-stranded RNA motifs was screened for the ability to modulate the specific IgG response to a protein antigen (OVA) administered via the respiratory tract. To simplify the process, the screening was organized into two rounds: (1) round one involving pools of RNA species (Table 1); and, (2) the second round dissecting components within pools endowed with maximal impact on the immune response.

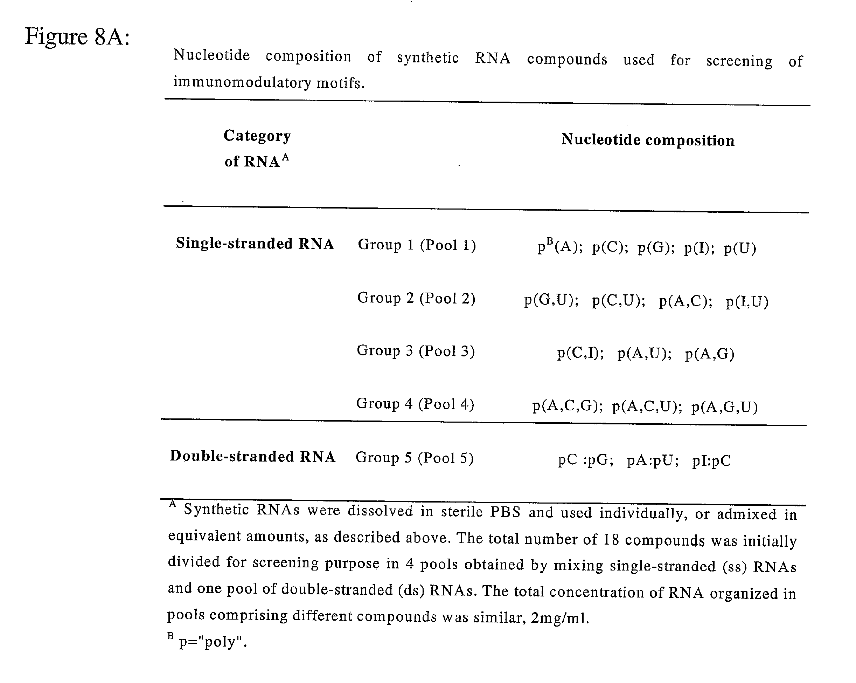

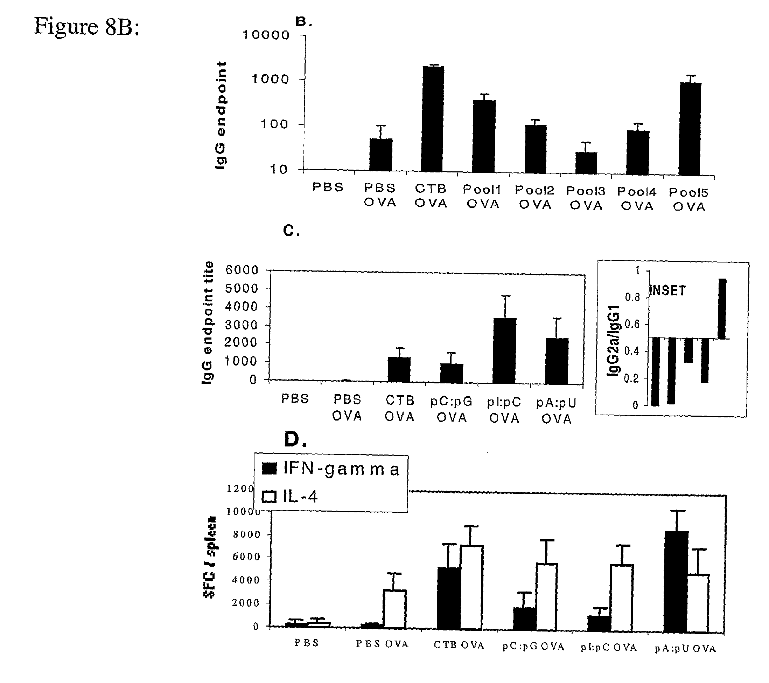

The double stranded RNA (dsRNA) or single stranded RNA (ssRNA) of the present invention is made by Sigma according to the following method:
ssRNA: The polynucleotides (polyA, polyU) are enzymatically prepared, using nucleotides and polynucleotide-phosphorylase, with no animal-sourced material entering into its preparation process. dsRNA: Annealing of polyadenylic acid (polyA, pA) with polyuridylic acid (polyU, pU). In general, the dsRNA and ssRNA of the present invention are homopolymers with, in the case of dsRNA, a single base or nucleotide (e.g., adenine)consistently forming one strand with its complement consistently forming the other strand. In the case of ssRNA, the single strand is consistently made of the same nucleotide. However, it is within the scope of the invention to use dsRNA or ssRNA compositions that are made up of mixed nucleotides (and their complements in the case of dsRNA). The dsRNA and ssRNA compositions of the present invention are comprised of the bases/nucleotides adenine (A), guanine (G), cytosine (C), uracil (U) and inosine (I). The RNA compositions in Table I and Figure 8A is descriptive of various RNA compositions used in the examples. The RNA compositions of the present invention were prepared and purified according to Example 27.

The various RNA strands used in the present invention are generally between 100 - 2000 base pairs in length but may be between 1 - 20, 20 - 40, 40 - 60, 60 - 80, 80 - 100, 1 - 100, 100 - 200, 200 - 300, 300 - 400, 400 - 500, 500 - 600, 600 - 700, 800 - 900, 1000 - 1100, 1100 - 1200, 1200 - 1300, 1300 - 1400, 1400 - 1500, 1500 - 1600, 1600 - 1700, 1700 - 1800, 1800 - 1900, 1900 - 2000, 2000 - 2100, 2100 - 2200, 2300 - 2400, 2400 - 2500, 2500 - 3000, 3000 - 4000, 4000 - 5000, 5000 - 10,000 base pairs and greater than 10,000 base pairs in length and/or mixtures thereof.

### Ex. 1: Impact of various pools of RNA motifs on antibody response to a standard antigen (OVA)

The impact of various RNA pools (Table 1) on the adaptive immunity was measured in C57B1/6 mice co-immunized with OVA via the respiratory tract. As described in the "Materials and Methods" section, the antibody response was expressed as mean ± SEM of IgG endpoint titers (n=4/group). As controls, OVA in sterile PBS, OVA with cholera toxin subunit B (CTB) and PBS alone, respectively. As shown in the Fig. 1A, the pool corresponding to dsRNA displayed maximal impact on the antibody response, with substantial enhancement of the specific immunity. In addition, mixtures of single-stranded species that are complementary to each other partially reproduced this enhancement.

This shows that both the nature of residues and secondary structure of RNA determine their ability to act as "danger" motifs, with effect on the specific B cell response.

### Ex. 2: Effect of various individual dsRNA motifs on the induction of antibody response to OVA

Experiments were carried out as in the previous example and as described in the "Materials and Methods" section, but instead of pools of RNAs, we used individual dsRNAs. The results are expressed in Figure 1B in the same manner as in Fig. 1A. The results are representative for two independent experiments. INSET: the ratio between mean IgG2a and IgG1 titers to OVA. The order from left to right is similar as in the main panel: PBS OVA, CTB OVA, pC:pG OVA, pI:pC OVA and pA:pU OVA. In this second round of screening as shown in Fig. 1B, it is shown that two categories of motifs organized as double-stranded RNA, namely pA:pU and pI:pC, have a significant impact on the development of IgG response to specific antigen in C57BL/6 mice. Similar results were obtained in TLR4-/- C₃H/HeJ mice that have impaired response to endotoxin (not shown).

Thus, individual RNA motifs display different ability to impact the antibody response in terms of magnitude and profile.

### Ex. 3: The magnitude and profile of T cell response induced by OVA together with various, individual dsRNA motifs.

The results obtained by ELISPOT analysis are expressed as mean ± SEM of the number of IFN-γ and IL-4 spot-forming-colonies (SFC) per spleen (n=4/group). As is shown in Figure 1C, both pA:pU and pI:pC (40 µg, see "Materials and Methods") had an amplifying effect on the specific T cell response. Surprisingly, the profile of T cell response to specific antigen was dependent on the nature of RNA residues, suggesting that the immune system is able to discriminate RNA-associated danger motifs. RNA motifs containing A and U residues, rather than I and C, were able to direct the differentiation of IFN-γ-producing Th1 cells (Fig. 1C) in C57BL/6 mice. This result reflected in differential induction of IgG isotypes, consistent with the Th profile (Fig. 1B - INSET).

In conclusion, different dsRNA motifs have different impact on the magnitude and profile of T helper response.

### Ex. 4: The effect of a defined dsRNA motif on the antibody response to a viral antigen, the HIV recombinant gp140 protein.

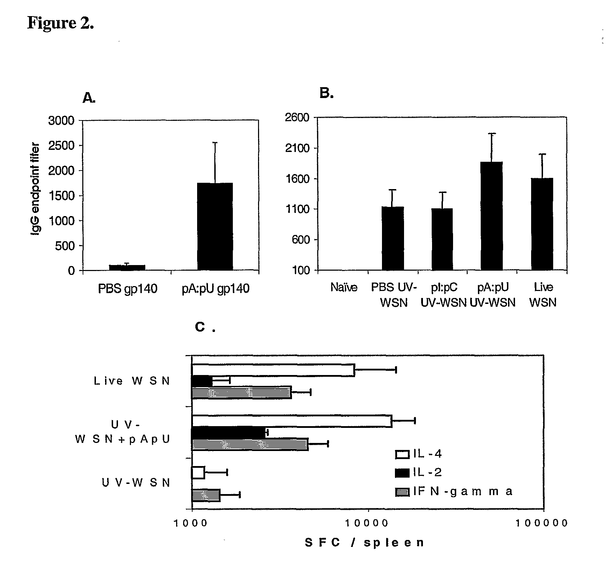

As shown in Figure 2A, the effect on the antibody response to the HIV gp140 (10 µg, see "Materials and Methods") fragment was measured in C57BL/6 mice immunized via the respiratory tract with antigen alone or antigen together with pA:pU (40 µg, see "Materials and Methods"). The results are expressed as mean ± SEM of IgG endpoint titers (n=3/group).

Thus, antibody responses to a viral antigen of potential practical use are enhanced by use of a novel dsRNA motif.

### Ex. 5: The effect of a defined dsRNA motif on the antibody response to whole UV-inactivated influenza virus, strain A/WSN/32 H1N1 (UV-WSN)

As in Example 4, the influenza virus-specific IgG antibodies were measured after mucosal immunization with UV-inactivated WSN virus (UV-WSN) (20 µg, see "Materials and Methods") alone or together with dsRNA motifs (50 µg). As a control, the antibody response subsequent to infection with the same strain of influenza virus (n=4/group) was used. The results are expressed as mean ± SEM of IgG endpoint titers in Figure 2B.

Thus, antibody responses to a viral antigen in context of whole inactivated microbe, are enhanced by use of a novel dsRNA motif.

### Ex. 6: The enhancement of T cell response to whole-inactivated influenza virus by co-administration of a defined dsRNA motif.

The T cell response to whole influenza virus was studied by ELISPOT analysis of splenocytes and expressed as IFN-γ, IL-4 and IL-2 SFC (mean ± SEM, n=4/group), after the subtraction of background. The T cell response to antigen together with pA:pU was compared to that subsequent to immunization with antigen alone or influenza virus infection (see Fig. 2C).

Thus as demonstrated in Examples 4 - 6 , the impact of dsRNA on antibody response was independently confirmed with foreign antigens, namely HIV envelope protein (recombinant gp140) and whole-inactivated influenza virus (Fig. 2A,B). In fact, pA:pU, rather than pI:pC, restored the titer of specific antibodies to influenza virus, to a level similar to that triggered by infection (Fig. 2B). In addition and rather significantly, pA:pU restored the T cell response to levels conferred by natural infection, in the context of immunization with inactivated virus (Fig. 2C). Thus, different RNA motifs exert a previously unknown, broad range of effects on T and B cell responses.

### 2) Motifs associated with dsRNA regulate the recruitment and activation of professional antigen presenting cells

It was hypothesized that dsRNA-associated danger motifs such as pA:pU and pI:pC influence the T cell response indirectly, via components of innate immunity. To test this hypothesis, the expression of chemokine genes was defined in the pulmonary lymphoid tissue, subsequent to administration of RNAs.

### Ex. 7: Local up-regulation of chemokine gene-expression by dsRNA motifs.

Local up-regulation of chemokine gene-expression by dsRNA motifs was measured by DNA array technique (see Materials and Methods "Measurement of Chemokine Gene Expression") one day subsequent to treatment via the respiratory tract. The results are expressed as fold-increase over expression levels measured in the pulmonary tissue of non-treated mice. The pattern of chemokine expression triggered by dsRNA was contrasted with that induced by LPS. The chemokines that selectively bind to receptors on Th1 and Th2 cells respectively, were indicated with continuous and interrupted contours (Fig. 3A).

The DNA array technique showed that IP-10, MIG, MIP-1α, MIP-1β and MCP-1 were strongly induced by both pA:pU and pI:pC (see Fig. 3A). However, only pI:pC triggered expression of RANTES, MCP-3 and CC chemokines that have the ability to engage receptors selectively expressed by Th2 cells. LPS induced a differential chemokine expression: upregulation of CXC chemokines MIG and MIP-1α, as well as the CC chemokine TCA-3 (see Fig. 3A). Thus, as previously unanticipated, a complex profile of chemokines is triggered by defined dsRNA motifs.

### Ex. 8: Recruitment of professional APC in the lungs of mice treated with dsRNA

Recruitment of professional APC in the lungs of mice treated with dsRNA motifs was assessed by FACS analysis one day after the treatment. The results are expressed in Figure 3B as percent CD11c⁺ and CD11b⁺ cells of the total cell population in the pulmonary interstitial tissue (n=4/group). CD11b⁺ (monocytes) were recruited by pA:pU and pI:pC, concordant with the upregulation of chemokine expression (see Ex. 7). In contrast, only pI:pC induced effectively a recruitment of CD11c⁺ dendritic cells. In conclusion, dsRNA motifs recruit APC at the site of administration.

### Ex. 9: Activation of professional APC (dendritic cells) by the dsRNA motifs.

Activation of professional APC by the dsRNA motifs was ascertained by *ex vivo* pulsing of CD11c⁺ cells with antigen together with dsRNA, followed by an adoptive transfer experiment into naïve BALB/c mice and measurement of T cell response (Fig. 3C). As controls, antigen-pulsed APC were used, or antigen-loaded cells co-stimulated with rIL-12 and anti-CD40 mAb, respectively. The results are expressed in Figure 3C, as number of IL-2 SFC estimated in the spleen by ELISPOT analysis.

Thus, dsRNA motifs activate APC in addition to the recruiting effect.

### Ex. 10: Cross-priming of MHC class I-restricted T cells against a viral antigen, stimulated by dsRNA motifs in BALB/c mice.

Cross-priming (referring to special circumstances when APC acquire the ability to prime class I restricted T cells without infection) stimulated by dsRNA motifs was studied in BALB/c mice treated with recombinant-engineered HIV gp140 antigen (10 µg) together with pA:pU, by ELISPOT analysis, using *in vitro* stimulation with MHC class I-restricted cognate peptide (see "Materials and Methods"). As a control, dose-matched gp140 antigen was used. The results are expressed in Figure 3D as mean ± SEM of the number of IFN-γ and IL-4 SFC / spleen (n=4/group).

In conclusion, dsRNA motifs facilitate the induction of MHC class I-restricted to non-infectious antigens of potential practical use.

### Ex. 11: Cross-priming of MHC class I-restricted T cells against OVA, stimulated by dsRNA motifs in C57BL/6 mice

Cross-priming stimulated by dsRNA motifs was studied in C57BL/6 mice treated with whole OVA together with pA:pU by ELISPOT analysis, using *in vitro* stimulation with MHC class I-restricted peptide (see "Materials and Methods"). As a control, dose-matched OVA antigen or sterile PBS was used. The results are expressed in Figure 3E as mean ± SEM of the number of IFN-γ and IL-4 SFC / spleen (n=4/group).

In conclusion, dsRNA motifs facilitate the induction of MHC class I-restricted to non-infectious antigens of potential practical use.

FACS analysis of pulmonary interstitial cells subsequent to mucosal administration of pA:pU and pI:pC showed prompt recruitment of CD11b⁺ monocytes and in the second case, of CD11c⁺ dendritic cells (Fig. 3B). In addition, *in vitro* incubation of CD11c⁺ DC from naïve mice with antigen together with pA:pU and to a lesser extent pI:pC, resulted in their activation since subsequent adoptive transfer of antigen-pulsed cells into BALB/c recipients, triggered enhanced class II-restricted T cell immunity (Fig. 3C). Similar enhancement has been measured by APC incubation with anti-CD40 antibody or IL-12. Finally, pA:pU had a strong CD8⁺ T cell-promoting effect in context of mucosal vaccination with recombinant HIV envelope protein (gp140) or OVA (Fig. 3D-E), as revealed by ELISPOT analysis with characterized MHC class I-restricted peptides from the HIV envelope protein and OVA, respectively. Thus, dsRNA has the ability to trigger differentiation of professional APC to a stage compatible with cross-priming of MHC class I-restricted T cells. This type of immune response is usually encountered only in case of viral infections. Use of defined dsRNA motifs may obviate the need for live vaccine vectors that are associated with side effects due to vector replication. Together, these data show a profound impact of RNA motifs on elements of innate immunity that in turn has the ability to regulate the adaptive immune response.

### 3) dsRNA motifs block the induction of high-zone tolerance and trigger protective anti-viral immunity

Danger molecules participate to discriminate between innocuous antigens and antigens associated with infectious processes. In high doses, non-infectious purified protein antigens induce unresponsiveness or immunological tolerance. Central ways of achieving tolerance to self or innocuous antigens is "immunological ignorance" and "immunological tolerance". In the first scenario, antigens are not accessible to APC due to spatial segregation. In the second scenario, the antigens are accessible to APC, are internalized, processed and the resulting epitopes are presented in context of poor co-stimulation. The net outcome can be the induction of immune unresponsiveness or tolerance at the level of T cells. In the case of infection or immunological challenge, there are mechanisms that prevent "immunological ignorance" and "tolerance". Such mechanisms occur via inducing novel migration patterns for APC together with activation of expression of co-stimulatory molecules and proinflammatory chemokines and cytokines. The outcome will be strong immune response rather than ignorance or tolerance to any antigens to which the immune system is exposed in such circumstances defined by the presence of "danger-molecules." As an example, tumor-associated antigens are often ignored by immune effectors or presented in a toleragenic context. Means to restore the immune competence against such antigens have direct practical implications in anti-cancer therapy. In order to test the danger-signal competence of pA:pU and pI:pC motifs, the model of tolerance achieved by intravenous inoculation of hIgG was used.

### Ex. 12: dsRNA motifs prevent high-zone tolerance in mice injected with human IgG.

The mice were initially injected intravenously with a standard toleragenic dose (200µg) of hIgG alone (closed symbols) or together with pI:pC or pA:pU (40-50µg) (open symbols; see Fig. 4A) and subsequently boosted subcutaneously with an immunogenic dose (50µg) of hIgG emulsified in CFA. The titer of antibodies against hIgG was measured by ELISA at various intervals after the boost. As a control, mice immunized with hIgG in CFA were used and represented the maximal titer (interrupted line). The results are represented in Figure 4A as means ± SEM of endpoint titers (n=5/group).

The results demonstrate that co-inoculation of either pA:pU or pI:pC together with hIgG in saline prevented the induction of B cell unresponsiveness, as shown by antibody titers subsequent to boost with hIgG in CFA (Fig. 4A). The dsRNA-associated motifs could both induce higher primary response and rescue the secondary response to this prototype antigen. Similarly, the assessment of T cell profile revealed that IL-4 and IL-2 production were partially restored by co-administration of dsRNA.

### Ex. 13: dsRNA motifs display differential ability to mobilize immune defense against influenza virus infection.

It was postulated that danger motifs have the capability of rapidly mobilizing protective arms of immune defense. Thus, it was tested whether pA:pU and pI:pC have any impact on the evolution of primary infection with influenza virus.

Mice were treated via the respiratory route with either pI:pC, pA:pU or saline one day before and after pulmonary infection with a sublethal dose of influenza virus. On day 5, the virus titer in the lung tissue was estimated and represented as total infectious units / organ (means ± SEM; n=6/group; results representative of two independent studies in C₃H/HeJ TLR-4-/- and competent mice).

As shown in Fig. 4B, it is shown that mice treated with RNA motifs via the respiratory tract were infected with sublethal doses of influenza virus. Five days after infection, the pulmonary virus titers were quantified. Similar results were obtained in C57BL/6 and TLR4-/- C₃H/HeJ mice (not shown). Notably, the dsRNAs were effective in orchestrating an effective reduction of pulmonary virus titers. Surprisingly, pA:pU was considerably more effective than pI:pC in curbing the pulmonary virus titers which further underlines the ability of the immune system to discriminate dsRNA-associated danger motifs. Thus, in the absence of immune memory, dsRNA motifs are able to mobilize an effective primary response against viral infection.

### 4) Enhancement of immunity to protein antigens by co-encapsulation with RNA danger motifs

Since the immune response to non-formulated subunit vaccines and in general, to purified protein antigens is minimal, it was tested whether *in vivo* co-exposure of APC to antigen and dsRNA danger motifs within the same microstructure results in a more favorable outcome. To this aim, a prototype antigen (OVA) was coformulated with pA:pU or pI:pC in short chain lipid complexes ("SCL")(see "Materials and Methods") with biocompatible and immunologically inert phospholipids (e.g., dioctanoylphosphatidylcholine) and lactose, as excipients. Upon delivery to the respiratory tract of C57BL/6 mice with either OVA+pA:pU or pI:pC formulated in short-chain lipid complexes, antibody responses were measured that were considerably higher than those of mice immunized with non-formulated antigen in saline, or antigen formulated in SCL complexes devoid of dsRNA motifs (Fig. 5A).

### Ex. 14: Short chain lipid complexes loaded with model antigen (OVA) alone or together with dsRNA motifs.

Short chain lipid complexes composed of short-chain phospholipids and loaded with model antigen (OVA) alone or together with dsRNA motifs have been generated and tested in C57BL/6 mice as shown in Figure 5A. The antibody response was measured by ELISA and represented as means ± SEM (n=4 / group; data representative for two independent measurements) of IgG endpoint titers at 2 weeks after intratracheal immunization. As controls, OVA in PBS and OVA co-formulated with CTB (choleratoxin B) in short chain lipid complexes (dioctanoylphosphatidylcholine) were used. The results show that molecular complexes comprising antigen and dsRNA, that preserve immunological properties of such RNA motifs, can be generated and are of practical use.

### Example. 15: Local (lung) and systemic (splenic) T cell response in C57BL/6 mice to whole OVA antigen or class I-restricted dominant OVA peptide, subsequent to immunization with OVA co-formulated with dsRNA motifs.

Figure 5B illustrates the results of local (lung) and systemic (splenic) T cell response in C57BL/6 mice to whole OVA antigen or class I-restricted dominant OVA peptide measured in mice immunized with OVA in short chain lipid complexes (dioctanoylphosphatidylcholine) with or without pA:pU. The analysis was carried out by ELISPOT and the results expressed as IFN-γ SFC / organ (mean ± SEM; n=4 / group).

Interestingly, CTB had only a limited adjuvant effect in context of short chain lipid complex co-formulation. Consistent with previous results, induction of T1 immunity was measured only with pA:pU particles as shown in Fig. 5B but not pI:pC, which displayed only enhancement of T2 immunity (not shown). Further, the T cell response to pA:pU co-formulated antigen see ("Materials and Methods") displayed an important local component (Fig. 5B). Finally, using the well-defined class I-restricted SIINFEKL [Seq. I.D. No. 2] peptide, it was confirmed that pA:pU retains the ability of promoting cross-priming in conjunction with SCL complexes (Fig. 5B).

### Ex. 16: Systemic and local antibody response of Sprague-Dawley rats to mucosal vaccination with SC-lipid complexes loaded with model antigen (OVA) co-formulated with dsRNA motifs.

Rats were immunized with lipid-complexes co-formulated with OVA and dsRNA. As controls, SC-lipid complexes devoid of antigen, SCL complexes loaded with OVA but devoid of dsRNA motifs and dose-matched amounts of OVA in saline were used, respectively. The results are expressed as endpoint titers (means ± SEM; n=4/group) of OVA-specific antibodies measured by ELISA in serum (Fig. 5C) and bronchoalveolar lavage fluid on day 35 (Fig. 5 D), respectively.

A similar enhancement of the antibody response has been measured in case of Sprague-Dawley rats aerosolized with SCL complexes loaded with OVA together with pA:pU or pI:pC (Fig. 5C). Lower titers were achieved with SC-lipid complexes devoid of dsRNAs or OVA in saline. The analysis of mucosal antibody titers (Fig. 5D) revealed a similar profile.

Thus, co-formulation of RNA-associated danger motifs and protein antigen by using a novel spray-drying technology preserves the immunomodulatory properties of RNA motifs and results in substantial increase of the specific immune response, both locally and systemically.

### Example 17: Non-Replicating dsRNA motifs act as master switch for the adaptive (B and T cell) immune response

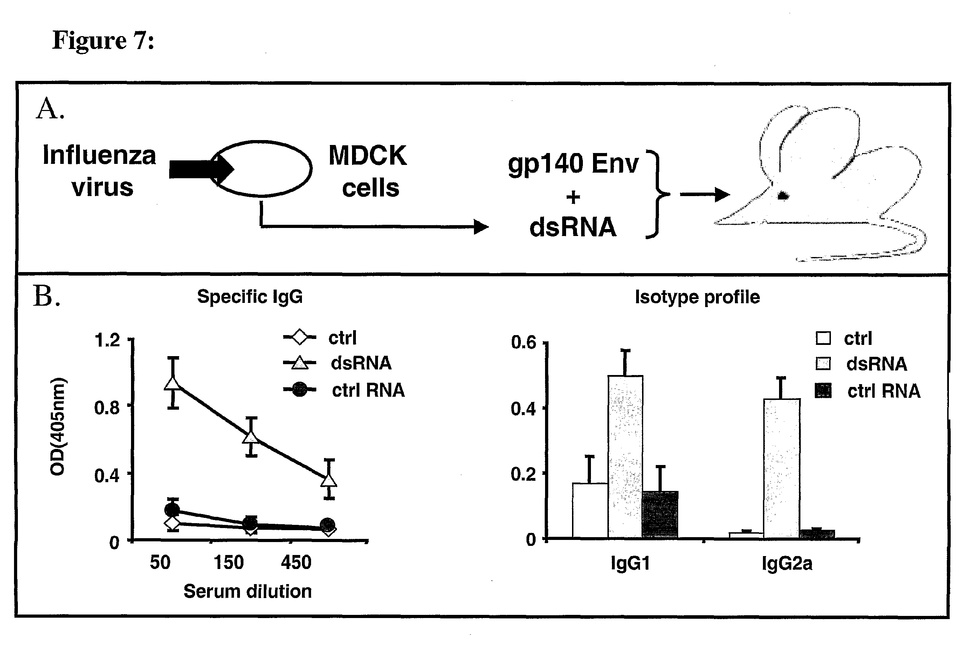

Antigens devoid of danger motifs such as dsRNA are poorly immunogenic or if provided in large doses may induce immunological tolerance. However, dsRNA motifs modify the way the immune system perceives the antigen: instead of poor responsiveness or tolerance, such motifs instruct the adaptive (T and B cell) immunity to mount strong responses to co-existing antigens, as well as prevent or block the immunological tolerance. Thus, innate immunity by virtue of recognition of dsRNA motifs operates as master switch for adaptive B and T cell immunity (Fig. 7).

### Example 18. Naturally occurring dsRNA bridges the innate with adaptive immune response. Example 18 shows that natural, non-infectious double stranded RNA produced during infection with influenza virus, has substantial effects on the specific immune response to a protein antigen.

Permissive MDCK cells were infected with WSN influenza virus (10⁸ TCID₅₀ / 1x10⁹ cells) and after 24 hours, the cells were harvested, washed and the total RNA extracted using an RNA separation kit (Qiagen, Valencia, CA). The RNA was further purified by treatment with RNAse-free DNAseI (Stratagene, San Diego, CA). The single stranded RNA in the samples was then removed by 30 minutes incubation at 37°C with 5µ of S1 nuclease (Ambion, Inc., Austin, TX) / µg of RNA. The RNA was analyzed prior to and subsequent to the digestion by gel electrophoresis. The absence of infectious properties of the purified dsRNA was confirmed by standard influenza virus titration. As a control, material purified and treated similarly, from 10⁹ non-infected MDCK cells was used. The concentration of nucleic acid was measured by spectrophotometry (A₂₆₀ₙₘ) and the absence of endotoxin confirmed by Limulus assay. The purified dsRNA and control RNA were used individually, or as a mixture with gp140 recombinant antigen (25µg of RNA and 2 µg of antigen in 25ml of sterile PBS).

After demonstrating lack of infectivity, 40µg of dsRNA or control RNA were admixed with 40µg of recombinant truncated antigen (gp140 of HIV envelope) and were administered to BALB/c mice by intranasal instillation (n=3/group). Additional controls were animals immunized with 40µg of gp140 protein in saline (n=3 / group). The mice were boosted once, at 2 weeks after priming. Blood was harvested 2 weeks after the boost, sera prepared and the antibody response against gp140 measured by ELISA. In brief, wells were coated with antigen (2µg/ml of gp140) and blocked with SeaBlock (Pierce, Rockford, IL, catalog # 37527). Serial dilutions of serum and bronchoalveolar lavage fluid were incubated for at least 2 hours at room temperature. After washing, the assay was developed with anti-mouse IgG antibody coupled with alkaline phosphatase (Sigma, cat# A7434) followed by addition of substrate (pNPP, Sigma, cat# N2765) and measurement by using an automatic microtiter plate reader (Molecular Devices, ThermoMax) equipped with SoftMax software.

In Fig. 7, panel A, the general principle of the experiment is illustrated. In Fig. 7, panel B, the absorption after assay development is represented, corresponding to various serum dilutions, in case of whole IgG. In Fig. 7, panel B, the absorption at 1/50 serum dilution, in case of IgG2a and IgG1 antibody isotypes, is represented.

Overall, the data in Fig. 7, panels A - B, show that natural, non-infectious dsRNA from influenza virus-infected MDCK cells, has an unexpected enhancing effect on the adaptive response to a prototype antigen. Both IgG1 and IgG2a antibody responses were increased, showing that a strong T helper1 and 2 response was induced.

### Example 19. Effects of selected RNA motifs on the innate immune response: heterogeneous motifs. This Example shows, unexpectedly, that different synthetic RNA motifs have a distinct effect on the adaptive specific immune response to a protein antigen.

Figure 8A shows an extensive library of synthetic RNA motifs, that was grouped in pools and used for a two-tier screening process as follows:
(A) The mice were immunized intratracheally with RNA pools, followed by 2 boosts two weeks apart, carried out by intranasal instillation. The antibody response measured (Fig. 8B) by ELISA was expressed as mean ± SEM of IgG endpoint titers (n=4/group). As controls, dose-matched OVA in sterile PBS was used, OVA with cholera toxin subunit B (CTB) and PBS alone, respectively. In brief, wells were coated with antigen (10µg/ml of OVA) and blocked with SeaBlock (Pierce, Rockford, IL, catalog # 37527). Serial dilutions of serum and bronchoalveolar lavage fluid were incubated for at least 2 hours at room temperature. After washing, the assay was developed with anti-mouse IgG antibody coupled with alkaline phosphatase (Sigma, cat# A7434) followed by addition of substrate (pNPP, Sigma, cat# N2765) and measurement by using an automatic microtiter plate reader (Molecular Devices, ThermoMax) equipped with SoftMax software.
(B) The effect of various dsRNA motifs on the induction of antibody response to OVA: the results are expressed as in Fig. 8C. The data are representative for two independent experiments. INSET: the ratio between mean IgG2a and IgG1 titers to OVA. For this purpose, biotin-conjugated anti-mouse IgG1 and IgG2a antibodies were used, followed by incubation with streptavidin-AKP conjugate. The order from left to right is similar as in the main panel in Fig. 8C: PBS OVA, CTB OVA, pC:pG OVA, pI:pC OVA and pA:pU OVA.
(C) The magnitude and profile of T cell response induced by OVA together with various dsRNA motifs, in female C57BL/6 mice. For the measurement of cellular response, splenic cell suspensions were obtained by passing the organ through 70 micron nylon Falcon strainers (Becton Dickinson, cat# 352350) followed by lysis of red blood cells with red blood cell lysis buffer (Sigma, cat# R7757). The lymphocytes from the pulmonary associated lymphoid tissue were isolated by collagenase (Sigma, cat# C9891) digestion of lung tissue followed by Ficoll-Paque (Amersham Pharmacia, cat# 17-1440-02) gradient centrifugation. The T cell response was measured by ELISPOT analysis as follows: 96-well 45 micron mixed cellulose ester plates (Millipore, cat#MAHA S4510) were coated with 4µg/ml of rat anti-mouse anti-IFNγ, IL-2 or IL-4 monoclonal antibodies (BD-PharMingen, cat#554430, cat#18161D, cat# 554387 respectively). After blocking with 10% FCS in sterile saline for 1 hour at 37°C, spleen cell suspensions were added at 5x10⁵ cells / well, with or without antigens / peptides. For stimulation, graded amounts of antigen (OVA) were used. At 72 hours after stimulation, the assay was developed with biotinylated rat anti-mouse cytokine antibodies (BD-PharMingen) followed by streptavidin-HRP (BioSource Int., Camarillo, CA) and insoluble ABC substrate. The results were measured using an automatic imaging system (Navitar/Micromate) equipped with multiparametric-analysis software (Image Pro, Media Cybernetics). The results are expressed in Figure 8D as mean ± SEM of the number of IFN-γ and IL-4 spot-forming-colonies (SFC) per spleen (n=4/group). The results are representative for two independent experiments.

The results in Figs. 8B - D show that different synthetic RNAs have an enhancing effect on the B and T cell response to a prototype protein antigen. In addition, different motifs, comprising specific nucleotide combinations, have specific effects in terms of T1 versus T2 induction and subsequently, immunoglobulin isotype switching.

### Example 20. Use of selected synthetic RNA motifs facilitates the induction of MHC class I-restricted Tc1 cells, producing IFN-γ.

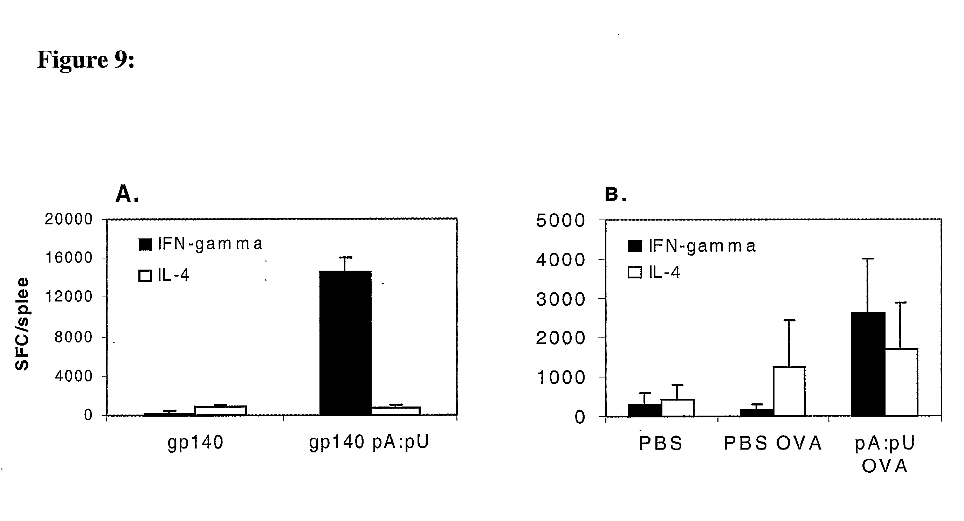

(A) Cross-priming stimulated by dsRNA motifs was studied in BALB/c mice treated (priming plus 2 boosts) with 10µg of recombinant-engineered HIV gp140 antigen together with pA:pU. The response was measured by ELISPOT analysis as described in Example 19, using *in vitro* stimulation with the MHC class I-restricted cognate peptide R10K derived from the V3 domain. As a control, dose-matched gp140 antigen was used. The results are expressed in Figure 9A as mean ± SEM of the number of IFN-γ and IL-4 SFC / spleen (n=4/group).

(B) Cross-priming stimulated by dsRNA motifs was studied in C57BL/6 mice treated with 100µg of whole OVA together with pA:pU by ELISPOT analysis as described in Example 19, using *in vitro* stimulation with the MHC class I-restricted peptide SIINFEKL [SEQ. I.D. No. 2]. As a control, dose-matched. OVA antigen in saline or sterile PBS was used. The results are expressed in Figure 9B as mean ± SEM of the number of IFN-γ and IL-4 SFC / spleen (n=4/group).

The results in Figures 9A - B show that a selected synthetic RNA motif was able to promote increased T cell immunity to different MHC class I-restricted peptides encompassed within larger antigens (polypeptides). This immune response comprised a Tc1 component, consisting in IFN-γ-producing MHC class I-restricted T cells.

Example 21 shows that unexpectedly, different synthetic RNA motifs bind to different cellular receptors; in other words, there are multiple receptors that discriminate among RNA motifs.

*In vitro* binding of CD11b⁺ APC, by fluorescently-tagged pA:pU, was measured by FACS analysis. The MACS-separated APC were incubated at 4°C for 30 minutes with 10µg/ml of tagged pA:pU ([pA:pU]-F), washed and analyzed. Alternatively, APC were preincubated for 10 minutes with 20 or 100µg/ml of non-tagged pA:pU, pA or pI:pC respectively, before staining with tagged pA:pU and FACS analysis. The profiles of stained (open area), non-stained (filled area) cells and the percentage of highly stained APC were represented in each panel, with logarithmic x axis. The data are representative of two independent measurements with 10,000 events acquired for each sample.

### Materials:

Mouse CD11b, CD11c Magnetic Separation Beads: Miltenyi Biotec, cat#130-049-601, cat#130-052-001 respectively;
ULYSIS Nucleic Acid Labeling Kit: Alexa 488, Molecular Probes cat#U21650; RNA Motifs:
   - pA:pU, (Sigma, Lot #22K4068);
   - pI:pC, (Sigma, Lot# 52K4047);
   - pA, (Sigma, Lot#22K4022);
FACS Buffer: PBS, 1% FCS, 0.1% sodium azide;
MACs buffer: PBS, 2mM EDTA, 0.5% BSA;
Collagenase Buffer: 0.225mg BSA, 0.0062mg collagenase in 50ml RPMI; and, 70um cell strainer: (Falcon / Becton Dickinson, cat#352350.

### Methods:

I Labeling of RNA Motifs:
   1. In the following protocol, each RNA motif was tagged with the ULYSIS Alexa 488 label.
II Splenocyte preparation:
   1. Isolate splenocytes and lung cells from 4 female C57 BL/6 mice;
      - Lung cells, in contrast to splenocytes, must be minced and incubated in collagenase buffer for 30 minutes at 37oC prior to the following step;
      - Pass through 70um falcon cell strainer;
      - Wash and resuspend in MACS buffer:
   2. Label with either CD11b or CD11c specific MACS beads following suggested protocol;
   3. Cells were then treated with:
      - Non-tagged pA, pA:pU, or pI:pC (20 or 100ug/ml) for 10 minutes at room temperature;
      - ULYSIS tagged pA or pA:pU was added at 1.5ug/tube and 10ug/tube, respectively, to match dye:dsRNA ratio of each motif.
   Mix and incubate 30 minutes on ice.
   Wash once and resuspend in FACS buffer
III Flow Cytometry:
   Run flow cytometric analysis to determine / compare competitive inhibition of tagged versus non-tagged RNA motifs and cell receptor binding.

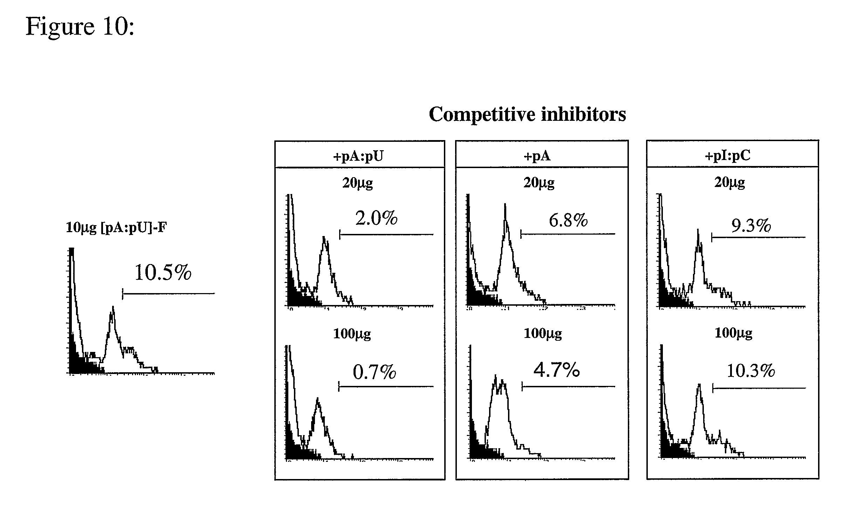

The results in Figure 10 show that pA:pU and pI:pC bind to different cellular receptors. Since pI:pC binds to TLR3, it demonstrates that additional receptors distinct from TLR3 are involved in RNA recognition immune function.

### Example 22 shows that selected synthetic RNA motifs trigger in vivo expression of chemokine genes, of importance for immunological activity.

Local up-regulation of chemokine gene-expression by dsRNA motifs was measured by DNA array technique using RNA from the pulmonary tissue, extracted one day after the administration via the respiratory tract. Total RNA was isolated from lungs using an RNeasy kit (Qiagen, Valencia, CA). The RNAs were further purified by treatment with RNase-free DNase I (Stratagene, San Diego, CA). DNA array was performed by using the Nonrad-GEArray kit from SuperArray Inc. (Bethesda, MD). Briefly, cDNA probes were synthesized using MMLV reverse transcriptase with dNTP mix containing biotin-16-dUTP. The GEArray membranes were prehybridized at 68°C for 1-2 hours. The hybridization was carried out by incubation of the membranes with biotin-labeled cDNA. The hybridized membranes were washed in 2xSSC - 1% SDS twice and 0.1xSSC - 0.5% SDS twice. The membranes were further incubated with alkaline phosphatase-conjugated streptavidin (BioSource Int., Camarillo, CA) and finally developed with CDP-Star chemiluminescent substrate. The intensity of signal was measured with Image-Pro analysis system equipped with Gel-Pro software (Media Cybernetics, Silver Springs, MD).

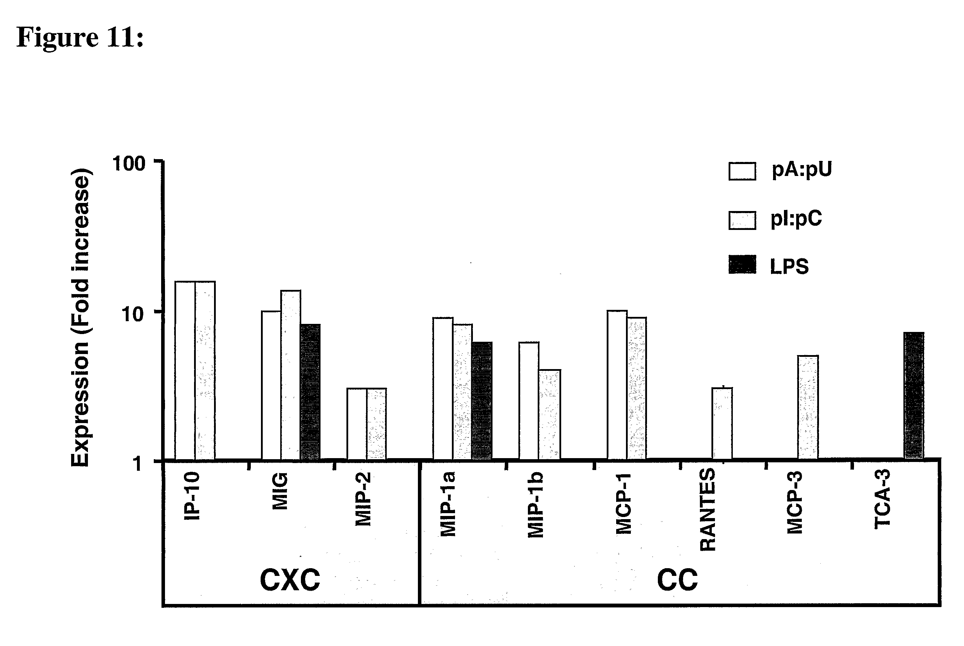

The results are expressed (Figure 11) as fold-increase of gene expression, over expression levels measured in the pulmonary tissue of non-treated mice. The pattern of chemokine expression triggered by dsRNAs (50 µg of pA:pU and pI:pC respectively) was compared to that induced by 1 µg of LPS. The chemokines that selectively bind to receptors on Th1 and Th2 cells were indicated with continuous and interrupted contours, respectively.

The results in Figure 11 show that pA:pU and pI:pC trigger expression of a wide range of chemokines, and that the expression pattern is motif-dependent and different from that elicited by LPS (endotoxin).

### Example 23 shows that selected synthetic RNA motifs mobilize an immune defense that is capable to control infection with a pulmonary virus.

dsRNA motifs display differential ability to mobilize immune defense against influenza virus infection. C3H/HeJ mice were treated via the respiratory route with 50µg of pI:pC, pA:pU or 50µl of saline one day before and after pulmonary infection with a sublethal dose of influenza virus. For virus challenge, C57BL/6 and TLR4-/- C₃H/HeJ mice under Metofane anesthesia were infected with sublethal doses (10⁴ tissue culture infective doses 50% - TCID₅₀) of live WSN (A/WSN/H1n1) virus, via the nasal route. On day 5 after infection, the mice were sacrificed, lungs retrieved, homogenized and stored at -70°C. The virus titers were measured by 48-hour incubation of serial dilutions of samples with permissive MDCK cells, followed by standard hemagglutination with chicken red blood cells (from Animal Technologies). The endpoint titers were estimated in triplicate measurements by interpolation and expressed as TCID₅₀ / organ (means ± SEM; n=6/group; results are representative of two independent studies in C₃H/HeJ TLR-4-/- and competent mice). Similar results were obtained in TLR4 competent, C57BL/6 mice.

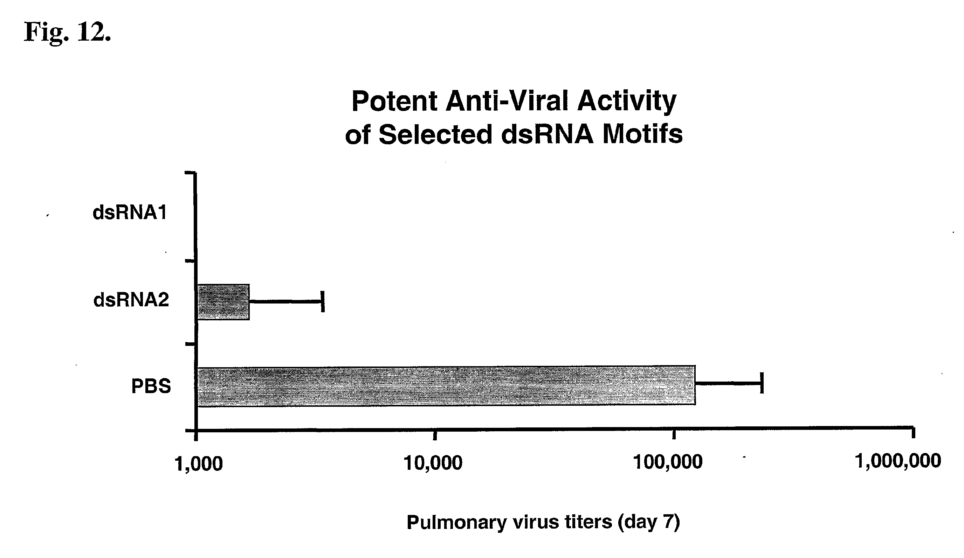

Thus, the results depicted in Figure 12 show that the control of replication of influenza virus can be achieved by using selected synthetic RNA motifs.

### Example 24 shows that co-administration of selected synthetic RNA motifs breaks tolerance to high dose standard antigen.

dsRNA motifs prevent high-zone tolerance in mice injected with human IgG. The mice (C57BL/6) were initially injected i.v. with a toleragenic dose of 200µg of hIgG alone (closed symbols) or together with 100µg of pI:pC or pA:pU (open symbols) and subsequently boosted subcutaneously with an immunogenic dose of 100µg of hIgG emulsified in CFA (Complete Freud's Adjuvant). The titer of antibodies against hIgG was measured by ELISA (as detailed in the Example 19), with the difference consisting in use of 10µg/ml of hIgG for coating) at various intervals after the first injection. As a control, mice immunized with 100µg of hIgG emulsified in CFA were included and represented the maximal titer on the graph (interrupted line).

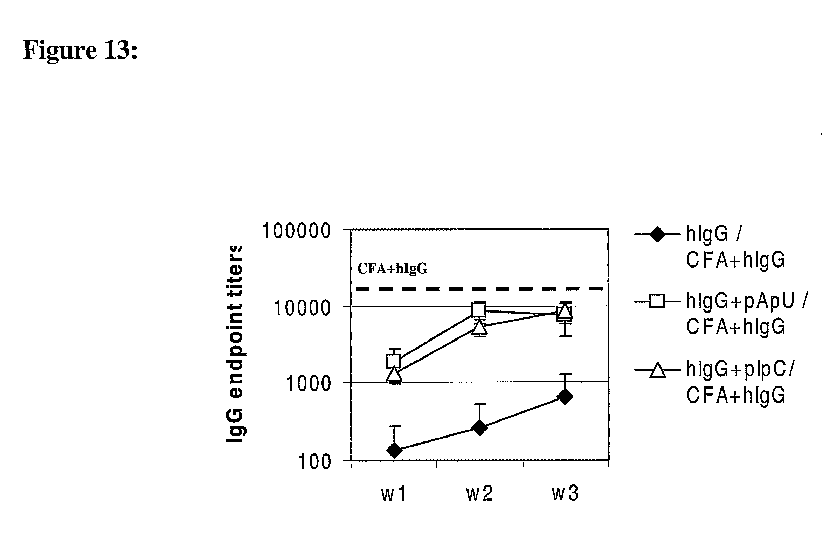

The results are represented in Figure 13 as means ± SEM of endpoint titers (n=5/group). Similar results were obtained in TLR4 deficient (C3H/HeJ) and LPS-responsive C3H/SnJ mice. Thus, the results in Figure 13 show that selected synthetic RNA motifs pI:pC and pA:pU largely prevent high zone tolerance that is usually associated with administration of large amounts of purified protein.

### Example 25 shows that selected RNA motifs induce differential cytokine production by human APC.

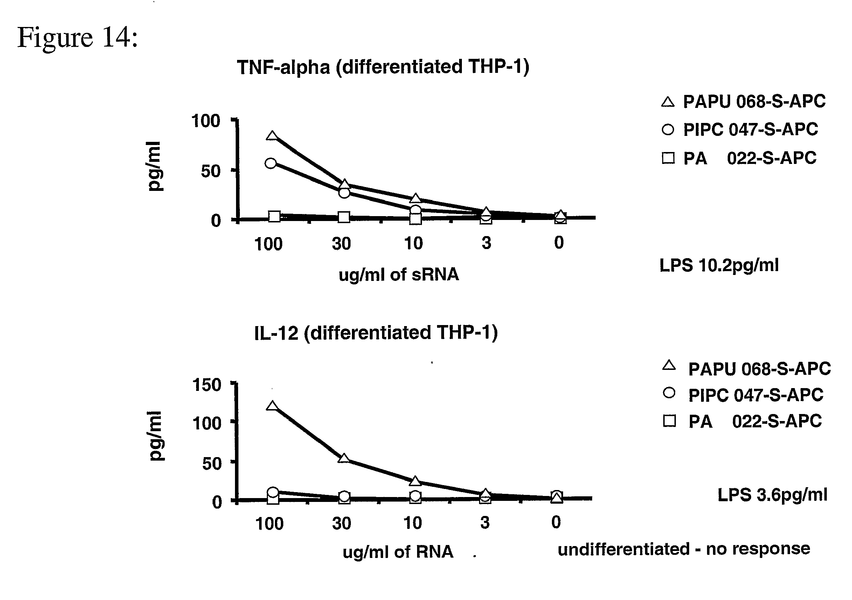

Human THP-1 monocytic cells, following differentiation, were incubated with different concentrations of synthetic RNA (pA:pU, pI:pC or pA) for 24 hours, and the cell supernatants collected. The concentration of IL-12 and TNF-α were measured by ELISA. The results are expressed in Figure 14 as pg/ml (concentration) for each cytokine and culture condition.

The results in Fig. 14 show that selected synthetic RNA motifs effect on human monocytic cells; in addition, this effect is heterogeneous, depending on the chemical structure of the motifs (nucleotide composition). Selected but not all synthetic RNA motifs are able to trigger IL-12 production, an important T1 regulatory cytokine, by human monocytic cells.

### Materials:

THP-1 Human monocytic cell line: ATCC, cat # TIB-202;
IL-12 Cytokine: Human ELISA, IL-12 ultra sensitive (US) cat# KHC0123;
TNF alpha Cytokine: Human ELISA, TNF alpha cat# KHC3012;
RNA Motifs:
   - pA:pU, (Sigma, Lot #22K4068);
   - pI:pC, (Sigma, Lot # 52K4047); and,
   - pA, (Sigma, Lot #22K4022).

### Method:

1. The THP-1 cells were allowed to differentiate following addition of 10ng/ml PMA in media containing 10% FCS.
2. After gently washing cells and adding non-FCS containing media (HL-1), treatments (RNA motifs and controls) were added at concentrations of from 3 to 100 µg/ml on top of adherent THP-1 cells.
3. After 24 hours incubation, cell supernatants were harvested and IL-12 and TNF alpha concentrations were measured by ELISA.

### Example 26 shows that two distinct synthetic RNA motifs bind to human THP-1 monocytic cells in a manner demonstrating interaction with different receptors.

THP-1 cells were incubated for 15 minutes at room temperature with different amounts of non-labeled synthetic RNA. Subsequently, tagged pA:pU was added for 30 minutes at 4°C, cells washed and the fluorescence quantified by FACS analysis.

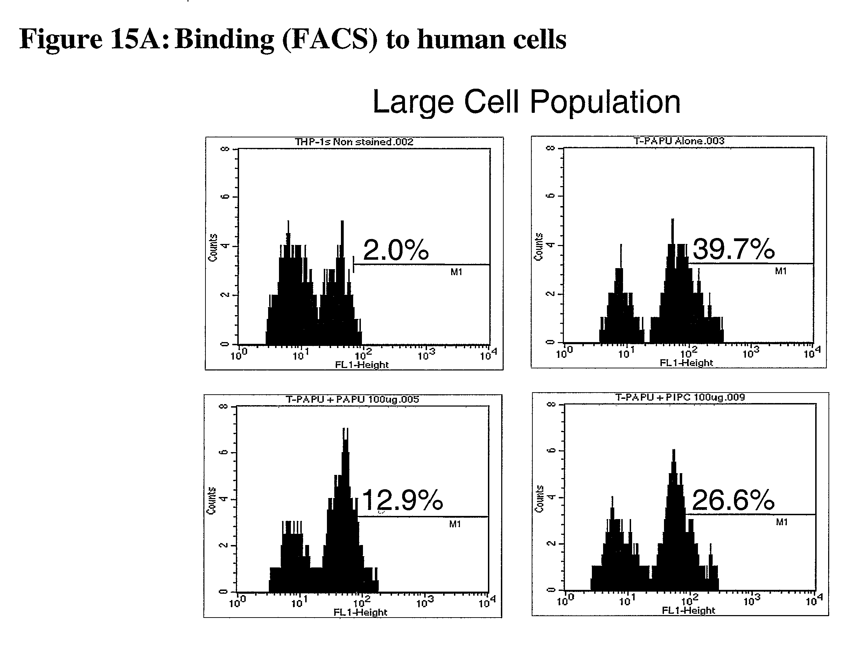

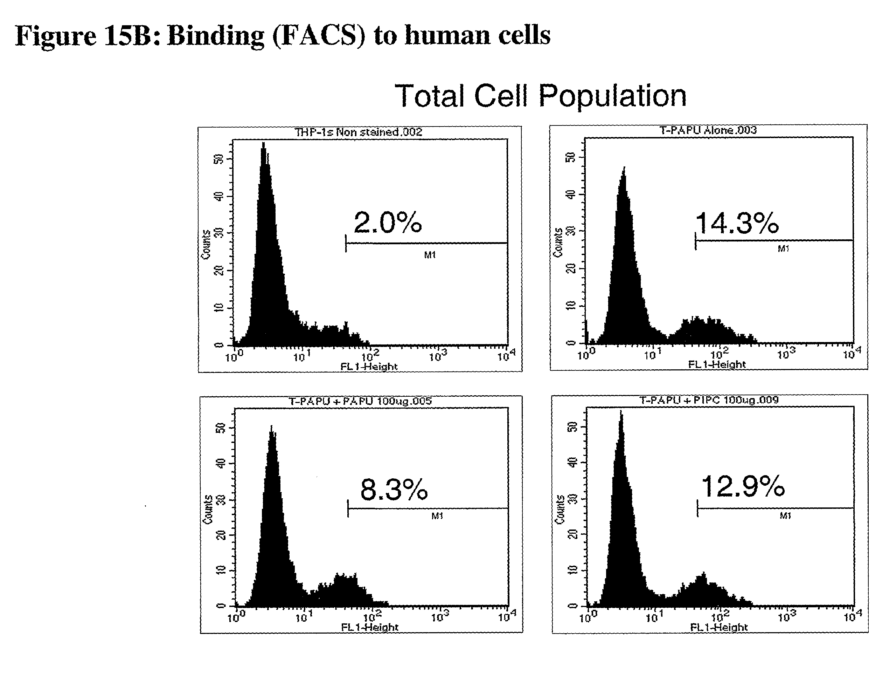

The results are expressed In Figs. 15A - 15B as histograms corresponding to the large cell subset (A) and total cell population (B). Percentages of stained cells were represented on each Figure.

The results in Figures 15A - 15B show that non-tagged pA:pU but not non-tagged pI:pC was able to compete out the binding of tagged pA:pU to human THP-1 monocytic cells, both at the level of large cell subset and whole population.

### Materials:

1. ULYSIS: Nucleic acid fluorescent label (Molecular Probes, cat# U-21650).
2. RNA Motifs:
   - pA:pU, (Sigma, Lot #22K4068);
   - pI:pC, (Sigma, Lot # 52K4047);
3. Detoxi-Gel column: (Pierce, cat#20344).

### Method:

I Labeling of Polyadenylic-Polyuridylic Acid (pA:pU):
   Following removal of endotoxin using a Detoxi-Gel column, pA:pU was labeled with the Alexa Fluor 488 fluorescent dye using the ULYSIS nucleic acid labeling system.
   Briefly:
      - The pA:pU was precipitated using sodium acetate and ethanol at -70°C;
      - The pA:pU was heat denatured and labeled with the Alexa Fluor 488 reagent at 90°C; and,
      - The reaction was stopped and the labeled pA:pU was ethanol precipitated.
II Cell treatment:
   THP-1 cells were suspended at 2X10⁶ cells /ml;
      50µl of above suspension (5X10⁴ cells) were placed in 12X75 mm tubes;
      Non-tagged pA:pU or pI:pC were added to the THP-1 cells at a concentration of either 20 or 100µg/ml and incubated 15 minutes;
      ULYSIS labeled pA:pU was added at a concentration of 100 ug/ml for 30 minutes on ice.

The THP-1 cells were washed once and suspended in FACS buffer followed by flowcytometric analysis to determine relative fluorescent differences between different treatment populations.

### Example 27 shows how the adjuvant synthetic RNA should be prepared and purified prior to use in its most effective format.

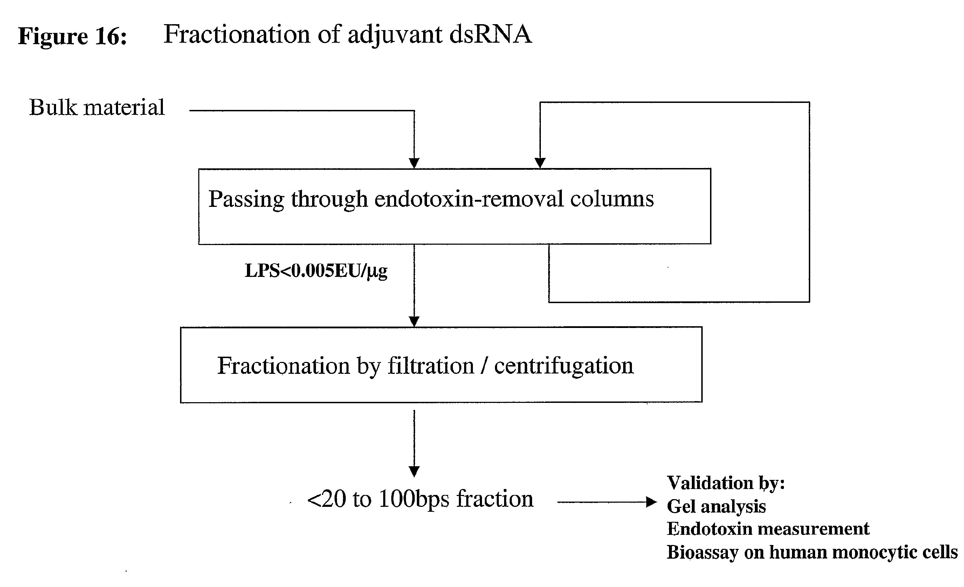

The bulk synthetic RNA material is obtained by standard methods of organic synthesis. Afterwards, the material is dissolved in sterile endotoxin-free saline, passed through endotoxin removal columns until the concentration of LPS is below 0.005EU/µg. The measurement of LPS is carried out by standard Limulus assay. Subsequently, the material is fractionated by a series of centrifugation steps through filters of defined porosity (see Fig. 16).

The useful fraction comprises synthetic RNA of less than 20 to maximum 100bp size. After purification, the material is measured and validated on standard assays: spectrophotometry (OD260nm); gel electrophoresis; endotoxin quantitation by Limulus assay; bioactivity on human THP-1 cells (as in Example 25).

### Example 28 shows that unexpectedly, different fractions of a selected synthetic RNA compound are endowed with different biological activity, based on size.

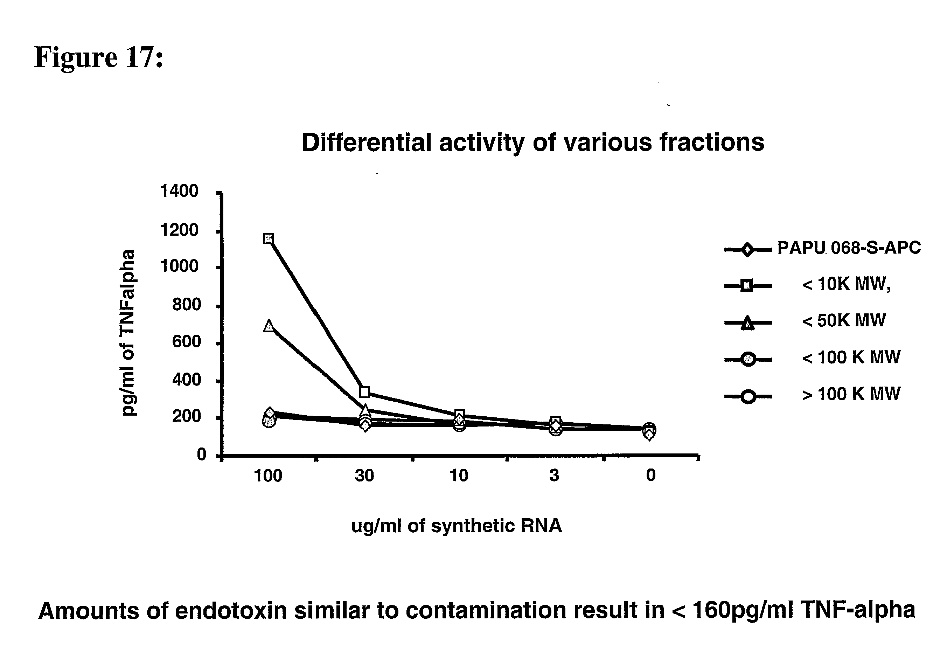

Differentiated human THP-1 monocytic cells were incubated with different concentrations of synthetic RNA (pA:pU, fractionated as described in the Example 27) for 24 hours, and the supernatants collected. The concentration of TNF-α was measured by ELISA using BioSource International kits (Camarillo, CA). The results are expressed in Figure 17 as pg/ml (concentration) for each culture condition.

The results depicted in Fig. 17 show that lower molecular weight fractions of a selected synthetic RNA compound are endowed with higher biological activity, in terms of cytokine production by human monocytic THP-1 cells.

### Example 29. Selected synthetic RNA motifs have, unexpectedly, a different immune profile in regard to generation of anti-RNA antibodies.

BALB/c mice were immunized intraperitoneally and subcutaneoulsy [i.p. + s.c.] with 50µg + 50µg of hIgG and synthetic RNA (pI:pC or pA:pU) and serum samples were prepared 1 week later. As a control, mice injected with hIgG in saline were used. The anti-hIgG, and dsRNA IgG antibody titers against pA:pU, pI:pC, pA and hIgG were measured by ELISA. In brief, wells were coated with antigen (10µg/ml of hIgG or synthetic RNAs) and blocked with SeaBlock (Pierce, Rockford, IL, catalog # 37527). Serial dilutions of serum and bronchoalveolar lavage fluid were incubated for at least 2 hours at room temperature. After washing, the assay was developed with anti-mouse IgG antibody coupled with alkaline phosphatase (Sigma, cat# A7434) followed by addition of substrate (pNPP, Sigma, cat# N2765) and measurement by using an automatic microtiter plate reader (Molecular Devices, ThermoMax) equipped with SoftMax software.

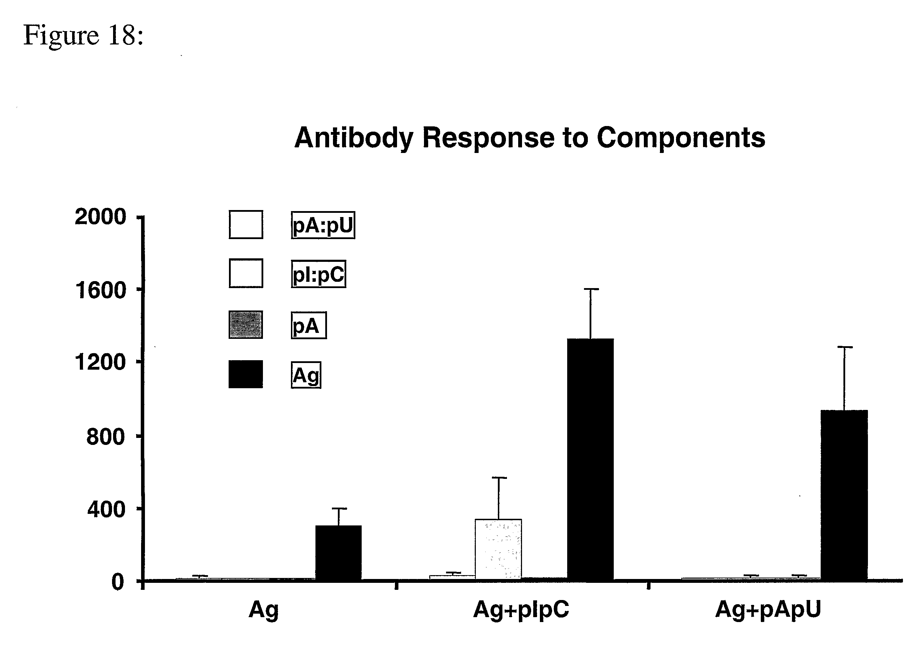

The results are expressed in Figure 18 as mean ± SEM of endpoint titers (n=3 / group). The results in Fig. 18 show that pI:pC but not pA:pU induced antibody response against itself, with a cross-reactive component against another RNA motif.

Example 30. In vivo loading of APC by recombinant IgG results in generation of Tc1 type of MHC class I responses only when additional conditions are satisfied.

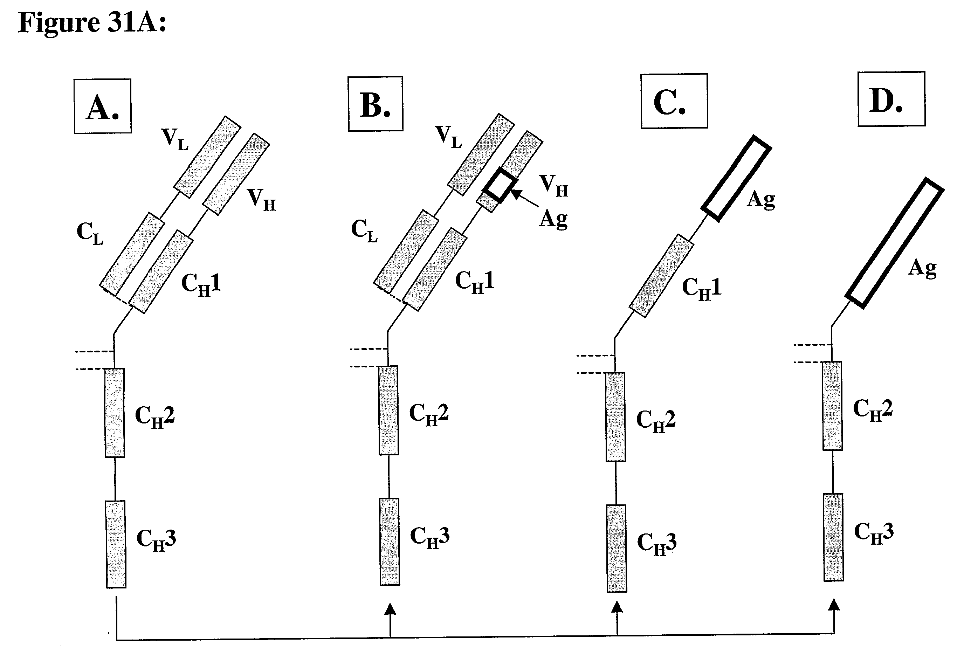

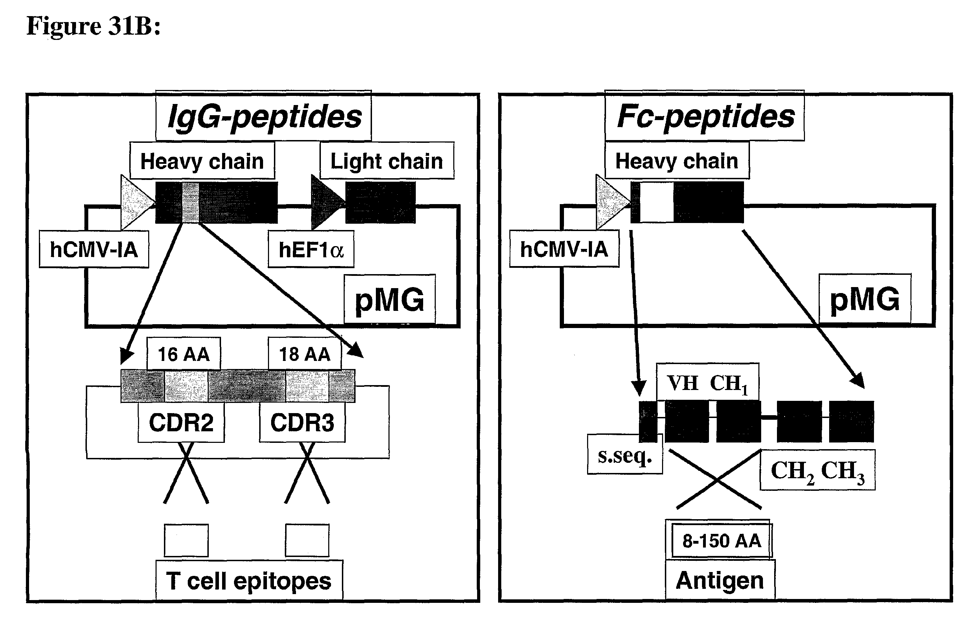

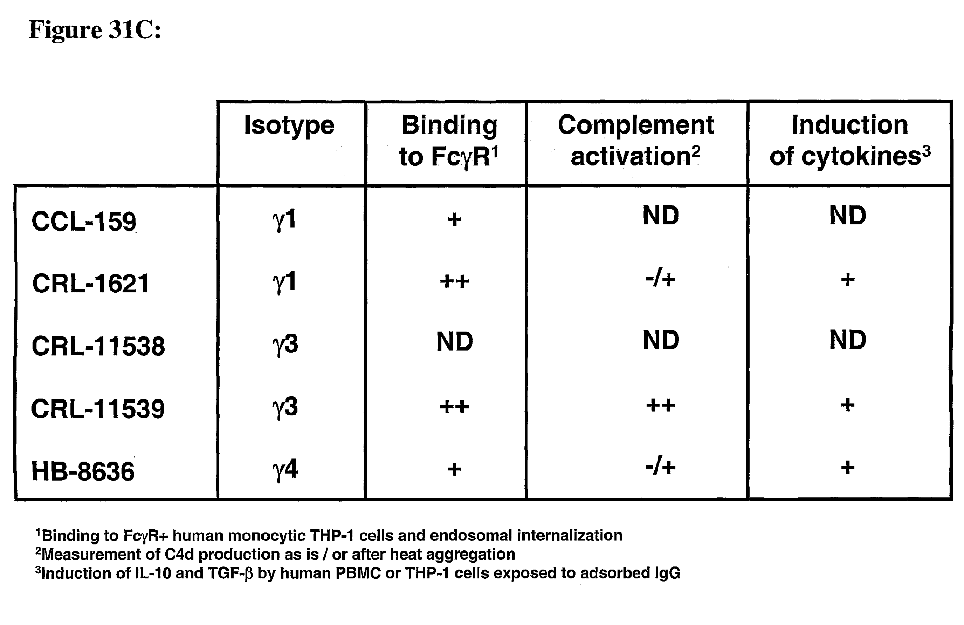

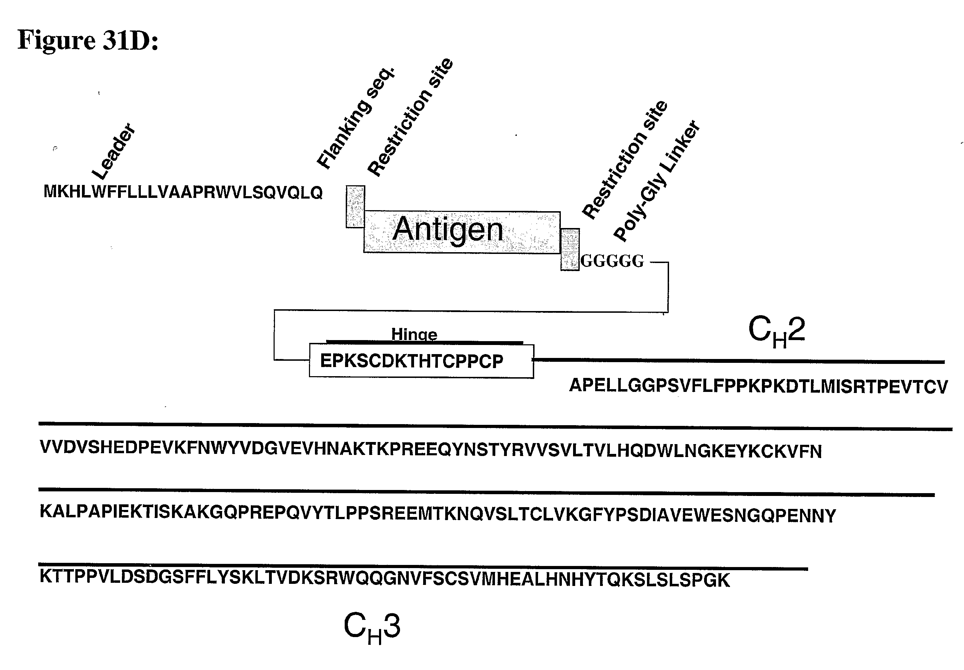

BALB/c mice were immunized with 50ug of recIgG-NP(Kd) (see Figs. 31A - 31D)(NP peptide is a protected and conserved epitope of Type A influenza viruses) subcutaneously, admixed with 50ug of selected synthetic RNA (pA:pU or pI:pC). As a control, naive mice or mice immunized with recIgG only were used. At 3 weeks after immunization, the T cell response was measured by ELISPOT analysis as follows: the ELISPOT plates (Millipore, Molsheim, France) were incubated with purified anti-cytokine Abs (4ug/ml for anti-IL4, and 8 µg/ml for anti-IFN gamma, from BD Pharmingen) in sterile PBS (50 µl/well) at 4° C overnight. The next day, the plates were washed 2 times with DMEM media and blocked with 200µl/well of DMEM complete containing FBS, for an hour at 37° C. Single cell suspension was made from the spleens, red blood cells were lysed, cells washed, counted and incubated at 5x 10⁵ /well together with NP 147-155 peptide or just with media, to assess the background. Plates were incubated 72 hours at 37 ° C, 5% CO2. After 3 days, plates were washed 5 times with PBS-tween20 0.05% (washing buffer), and incubated with 100 µl/well of biotinylated anti-cytokine Abs, 2 µg /ml in PBS- tween20 0.05% - FBS 0.1%(ELISPOT buffer) overnight at 4 ° C.

The next day plates were washed five times with washing buffer, and incubated for an hour with 1:1000 Streptavidin-HRP diluted in ELISPOT buffer. The reaction was developed with 3-amino-9-ethylcarbazole substrate (Sigma, St. Luis, MO) and stopped by washing the plate twice with tap water. Plates were then allowed to dry at room temperature for 24 hours.

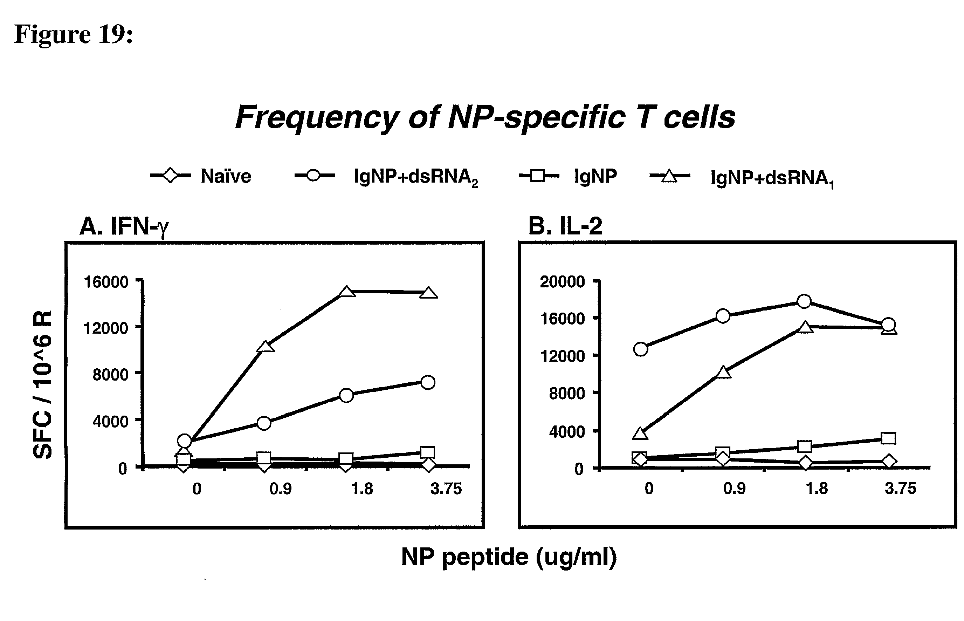

The data were acquired using an automated system (Navitar, Rochester, NY) with ImagePro-Plus) software (Media Cybernetics, Silver Spring, MD). The frequency of cytokine producing T cells reacting to NP peptide (NP peptide is a protective and conserved epitope of Type A influenza virus) was measured and expressed against the amount of peptide used for stimulation. The results are expressed in Fig. 19 as means + SEM of triplicates (n=3 mice / group).

The administration of recombinant IgG bearing the NP MHC class I-restricted epitope resulted in generation of Tc2 immunity but not Tc1 response, implying *in vivo* formation of class I-peptide complexes with a specific co-stimulation profile. The results in Fig. 19 show that co-use of selected synthetic RNAs promoted effective induction of IL-2 and IFN-gamma subsequent to IgG mediated delivery of an MHC class I-restricted epitope (dsRNA1 is pA:pU and dsRNA2 is pI:pC).

Example 31: Effective formation of MHC class 1-peptides and instruction of the resulting T cell response by simultaneous manipulation of APC loading via Fcgamma R and activation via RNA receptors.

Splenic APC were isolated from naive BALBc mice and pulsed *ex vivo* overnight with 1 ug NP peptide, or 50 µg recIgG-NP (Kd) with or without 50 µg/ml selected synthetic dsRNA (pA: pU). The cells were washed and 5x10⁶ cells were administered by s.c. and i.p. injection equal amount, to naive BALB/c mice. The response was measured 3 weeks later by ELISPOT analysis as follows: the ELISPOT plates (Millipore, Molsheim, France) were incubated with purified anti-cytokine Abs (4µg/ml for anti-IL4, and 8 µg/ml for anti-IFN gamma, from BD Pharmingen) in sterile PBS (50 µl/well) at 4° C overnight. The next day, the plates were washed 2 times with DMEM media and blocked with 200µl/well of DMEM complete containing FBS, for an hour at 37° C. Single cell suspension was made from the spleens, red blood cells were lysed, cells washed, counted and incubated at 5x 10⁵ /well together with 30 µg/ml ,10 µg /ml, or 3 µg /ml NP peptide or just with media, to assess the background. Plates were incubated 72 hours at 37 ° C, 5% CO2. After 3 days, plates were washed 5 times with PBS-tween20 0.05% (washing buffer), and incubated with 100 µl /well of biotinylated anti-cytokine Abs, 2 µg /ml in PBS- tween20 0.05% - FBS 0.1%(ELISPOT buffer) overnight at 4 ° C. The next day plates were washed five times with washing buffer, and incubated for an hour with 1:1000 Streptavidin-HRP diluted in ELISPOT buffer. The reaction was developed with 3-amino-9-ethylcarbazole substrate (Sigma, St. Luis, MO) and stopped by washing the plate twice with tap water. Plates were then allowed to dry at room temperature for 24 hours.

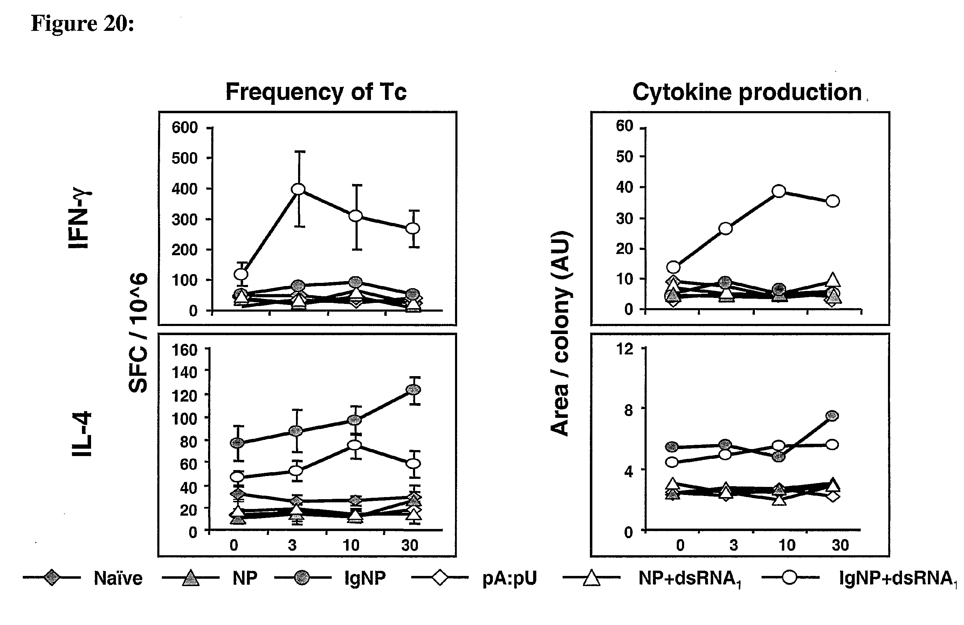

The data were acquired using an automated system (Navitar, Rochester, NY) with ImagePro-Plus) software (Media Cybernetics, Silver Spring, MD). The results are expressed in Figure 20 as frequency of cytokine producing spot forming colonies against the concentration of peptide used for *ex vivo* stimulation (mean ± EM, n=3 mice /group). In addition, the mean area /colony versus the concentration of peptide used for stimulation is plotted, for both IFN-gamma and IL-4 (arbitrary units).

The results in Fig. 20 show that *ex vivo* APC loading by recombinant IgG is more effective in formation of MHC class I-peptide complexes and generation of Tc response, compared to use of peptide itself. In addition, the mere formation of MHC class I-peptide complexes subsequent to epitope delivery via IgG / FcgammaR results in differentiation of Tc2 cells producing IL-4 but not IFN-gamma. Simultaneous treatment of APC with selected synthetic RNA results in broadening of the T cell profile, to IFN-gamma producing Tc1 cells.

### Example 32 shows that co-priming with IgG-peptide together with a selected co-stimulatory motif resulted in more effective secondary expansion of MHC class I-restricted T cells subsequent of virus infection.

BALB/c mice were injected with recIgG-NP(Kd), pA:pU separately, or in combination ( 50 ug / injection). As a control, naive mice were used. Three weeks after treatment, the mice were infected with 104 TCID50 of A/WSN/32 H1N1 influenza virus, via the respiratory tract. Four days after infection, the T cell profile in spleen was measured by ELISPOT analysis subsequent to *ex vivo* stimulation with NP peptide as follows: the ELISPOT plates (Millipore, Molsheim, France) were incubated with purified anti-cytokine Abs (4ug/ml for anti-EL2 and anti-IL4, and 8 µg/ml for anti-IFN gamma, from BD Pharmingen) in sterile PBS (50 µl/well) at 4° C overnight. Next day, the plates were washed 2 times with DMEM media and blocked with 200µl/well of DMEM complete containing FBS, for an hour at 37° C. Single cell suspension was made from the spleens, red blood cells were lysed, cells washed, counted and incubated at 5x 10⁵ /well together with 20 µg /ml NP peptide or just with media, to assess the background. Plates were incubated 72 hours at 37 ° C, 5% CO2. After 3 days, plates were washed 5 times with PBS-tween20 0.05% (washing buffer), and incubated with 100 µl /well of biotinylated anti-cytokine Abs, 2 µg /ml in PBS- tween20 0.05% - FBS 0.1%(ELISPOT buffer) overnight at 4 ° . The next day plates were washed five times with washing buffer, and incubated for an hour with 1:1000 Streptavidin-HRP diluted in ELISPOT buffer. The reaction was developed with 3-amino-9-ethylcarbazole substrate (Sigma, St. Luis, MO) and stopped by washing the plate twice with tap water. Plates were then allowed to dry at room temperature for 24 hours.

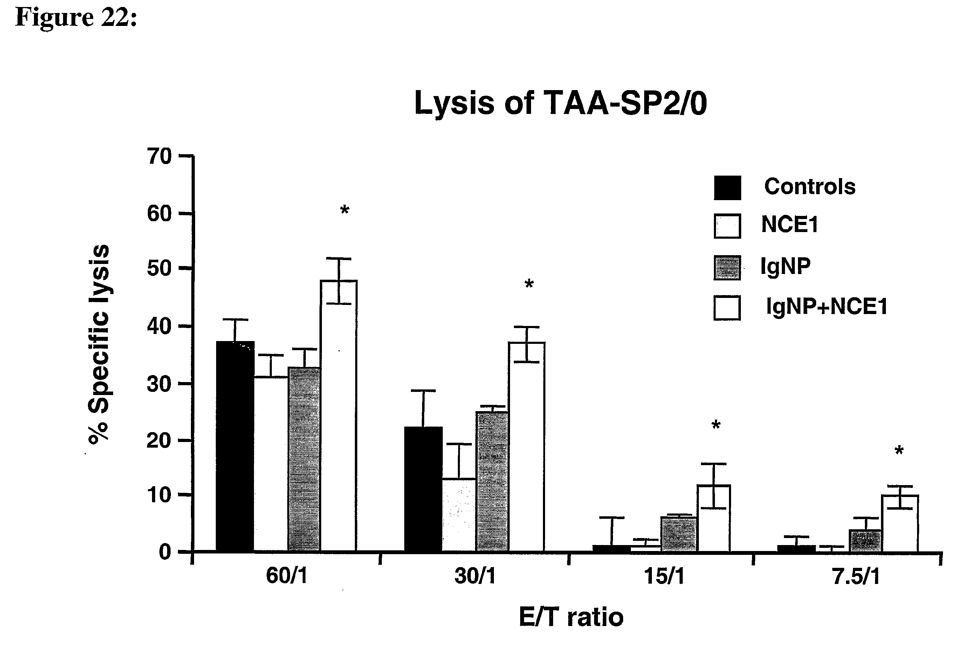

The data were acquired using an automated system (Navitar, Rochester, NY) with ImagePro-Plus) software (Media Cybernetics, Silver Spring, MD). The results are expressed in Figure 22 as frequency of NP-specific MHC class I-restricted T cells forming cytokine producing colonies (means ± SEM, n=4 mice / group).

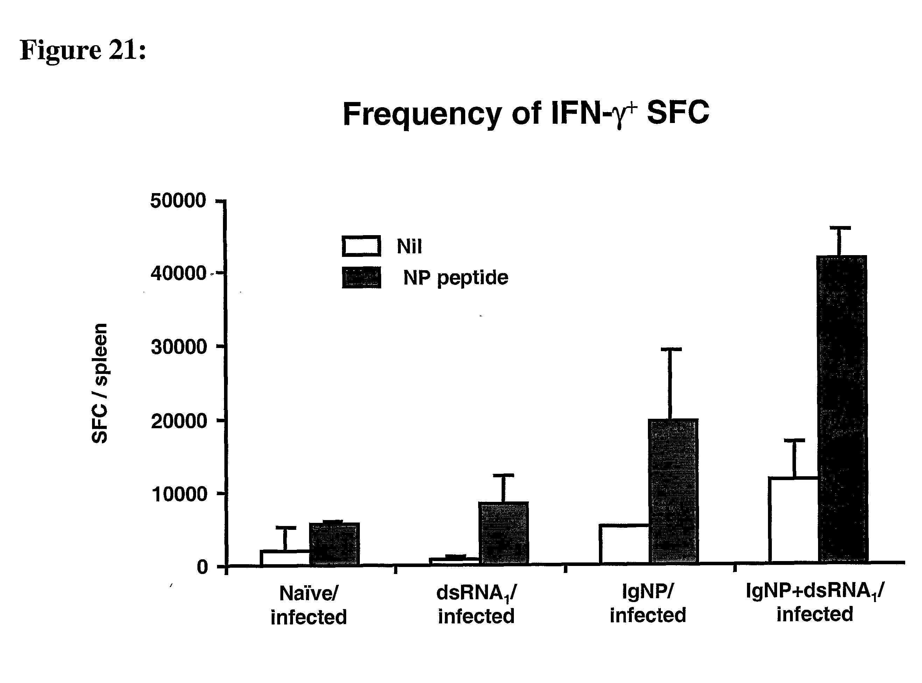

The results in Fig. 21 show that IgG mediated delivery of a class I restricted epitope is most effective in priming class I restricted Tc1 responses when co-administration of selected synthetic RNA was carried out. Such primed precursors were rapidly expanded subsequent to infection with influenza virus.

### Example 33 shows that the most effective priming of cytotoxic lymphocytes recognizing an MHC class I-restricted epitope occurs by co-administration of selected RNA motif together with peptide epitope inserted within the IgG backbone.

BALBc mice were immunized and challenged with recIgG-NP (Kd) as in the previous Example, and sacrificed 4 days after influenza virus infection. The splenocytes were prepared, suspended in HL-1 medium at 5 million / ml and co-incubated for 5 days with 10µg/ml of NP 147-155 peptide and in presence of 5U/ml of recombinant IL-2. Splenocytes from 4 mice / group were pooled and incubated in flasks.

After expansion, viable cells were recovered by Ficoll gradient centrifugation, washed and incubated for 5 hours in V-bottom plates, in various numbers, with a fixed number of sp20 target cells with or without NP peptide (20µg/ml). The supernatants were harvested after plate centrifugation, and the concentration of LDH measured by using a Promega kit (cat # G1780). The results are expressed as percent specific lysis at different E: T ratios (Effector to Target ratio).

The results in Fig. 22 show that effective priming of anti-viral cytotoxic T cells requires both effective *in vivo* loading of APC with class I restricted epitope delivered via IgG, together with appropriate instruction by selected synthetic RNA motif, namely pA:pU.

### Example 34 shows that vaccination with an IgG bearing a viral MHC class I-restricted epitope, together with selected synthetic RNA motif, provided protection against infectious challenge with a prototype virus.

BALB/c mice were immunized with 50ug of recIgG-NP (Kd) together with 50ug of selected synthetic RNA (pA: pU), by subcutaneous injection. Three weeks after immunization, the mice were challenged with 10⁴ TCID 50 of infectious WSN influenza virus and sacrificed 5 days later. The pulmonary virus was titrated in lung homogenates by standard MDCK hemagglutination assay as follows: on day one MDCK cells were plated in 96 well plates at 2x10⁴ / well/ 200ul and incubated for 24 hours at 37° C, 5% CO₂. Next day, 25 µl of the 10 fold dilutions in DMEM media of the lung homogenates were incubates in briefly tripsinized MDCK plates (1 minute) in triplicates and incubated at 37° C. After one hour 175 ul of the DMEM complete media was added and plates were incubated for 48 hours at 37° C, 5% CO₂. After two days the hemagglutination-inhibition was done with chicken red blood cells incubated with the cell culture supernatants from the MDCK plate for 30 minutes at room temperature and the results were expressed as means ± SEM of total pulmonary virus (n=4 mice / group). As a control, we used non-immunized mice.

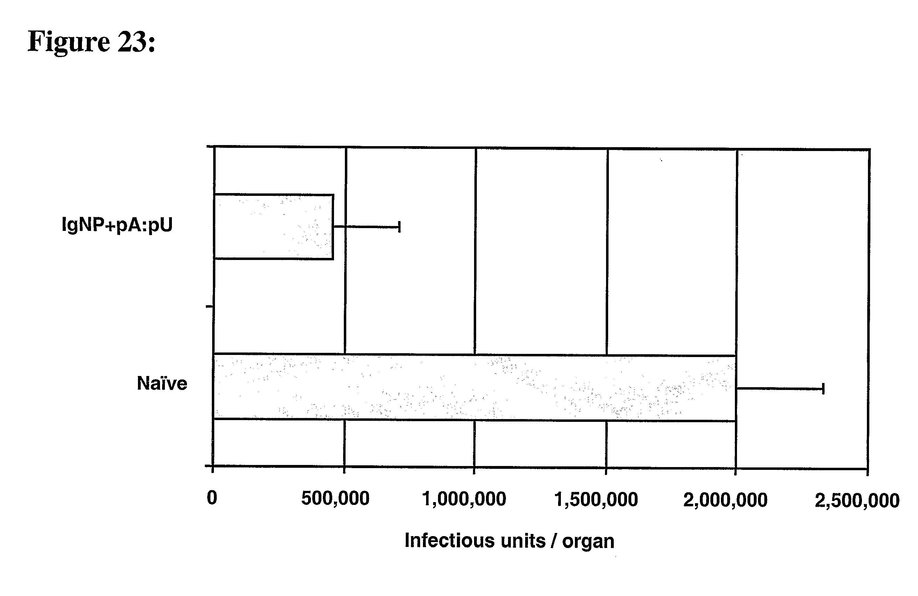

The results in Fig. 23 show that immunization with a recombinant IgG bearing a viral class I restricted epitope together with selected synthetic dsRNA, resulted in priming of an immune response capable to limit the replication of a virus subsequent to infectious challenge.

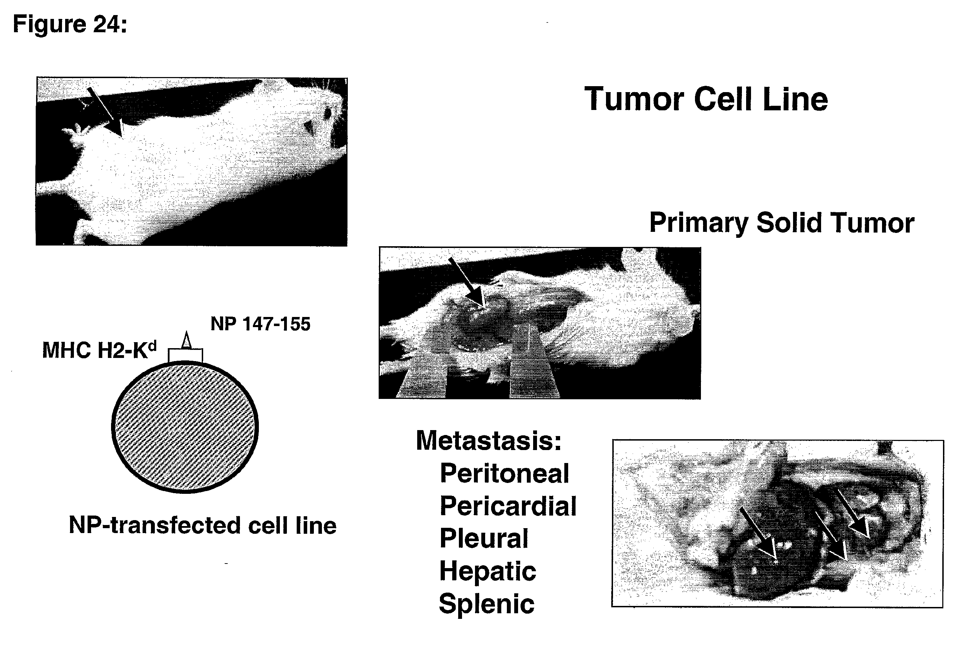

### Example 35. Figure 24 describes the tumor models used for testing the efficiency of a Ig-peptide-based molecules.

Balb-c mice (K^{d} restricted) have been used to establish a tumor model. Tumor cells (1 to 15 million in 100 µL) were typically injected in the flank (see arrow in upper photo). Primary tumors (i.e. those at the sight of injection) were first detected by palpating the area and then quantitated by measuring the tumor size with a caliper. In one series of experiments the mouse myeloma cell line (SP2/0), either untransfected cells or cells stable transfected expressing heterologous protein (recombinant IgG expressing different epitope peptides in the CDR3 region of the heavy chain or the complete NP protein), was used to induce tumors in the mice. Expression of heterologous proteins in the SP2/0 cells provided specific tumor associated antigens (TAA) for testing various anti-tumor strategies in the immunocompetent mice. Typically, untreated mice developed palpable solid primary tumors 1 week post injection that led to morbidity and death over the next 4 weeks. Postmortem examination of the injected mice revealed metastatic lesions (see Figure 24). Sp2/0 cells were cultured from primary tumor tissue as well as spleen taken from tumor-bearing mice (data not shown). SP2/0 cells were stably transfected with a recombinant IgG-expressing plasmids that were all identical except for the specific epitope sequence introduced into the CDR3 region of the heavy chain, for example the MHC I restricted NP epitope (amino acids 147-155). SP2/0 cells were also stably transfected with a plasmid containing the coding sequence for the entire NP protein of WSN virus under control of the CMV promoter. All transfected cell lines produced primary tumors over the same frame as wild type SP2/0 cells.

This tumor model was extended to include an adenocarcinoma cell line (4T1, ATCC CRL-2539, K^{d} restricted) previously shown to induce metastatic tumors in Balb-c mice. The 4T-1 cell line was similarly to that described above for the SP/0 line. Injection of 1 to 15 million 4T-1 cells into the flank of Balb-c mice produced a palpable primary tumor over a time frame similar to injections of SP2/0 cells eventually leading to death. Postmortem collection of tissue from various organs showed that 4T-1 could be recovered from spleen, lungs as well as the primary tumor (not shown). 4T-1 cells were stably transfected with a NP-expressing plasmid described above. As with SP2/0 cells, transfection of the 4T-1 cell did not affect the course of tumor growth and lethality of disease.

### Example 36 demonstrates successful control and treatment of a tumor after clinical diagnosis, by using a tumor associate T cell epitope within a recombinant IgG, together with a selected co-stimulatory RNA motif.

Balb/c mice were injected with SP2/0 cells (15 million in 100 µL) stably expressing recombinant IgG carrying the MHC I (Kd) NP epitope peptide in the CDR3 region of the heavy chain (IgNP). At day 7 post injection all mice had palpable tumors and the mice were randomized into 3 groups: co-stimulatory motif (i.e. dsRNA comprised of polymeric pApU) alone; purified IgTAA protein (IgNP); both. The time of treatment is indicated by the arrows, and each injection contained 50 µg of the indicated compound. The mice that developed metastatic disease and died are represented with a "D" in the figure.

The data show that the combination of dsRNA (co-stimulatory motif) and IgTAA (IgNP) produced a dramatic protective response in mice that all had primary tumors at the start of therapy. While all mice treated with either compound alone succumbed to disease, 100% of the mice treated with both were still alive 3 weeks after initiation of treatment and they were in good clinical condition at the time of sacrifice for measurement of T cell response. These data show that *in vivo* loading of APC with TAA (accomplished by uptake of IgNP via the Fc receptor of APC) is not sufficient for a potent anti-tumor response. The tumor rejection and survival displayed by mice treated with IgNP in combination with pApU dsRNA highlights the important role co-stimulation plays in treatment of tumors with tumor-associated antigens.

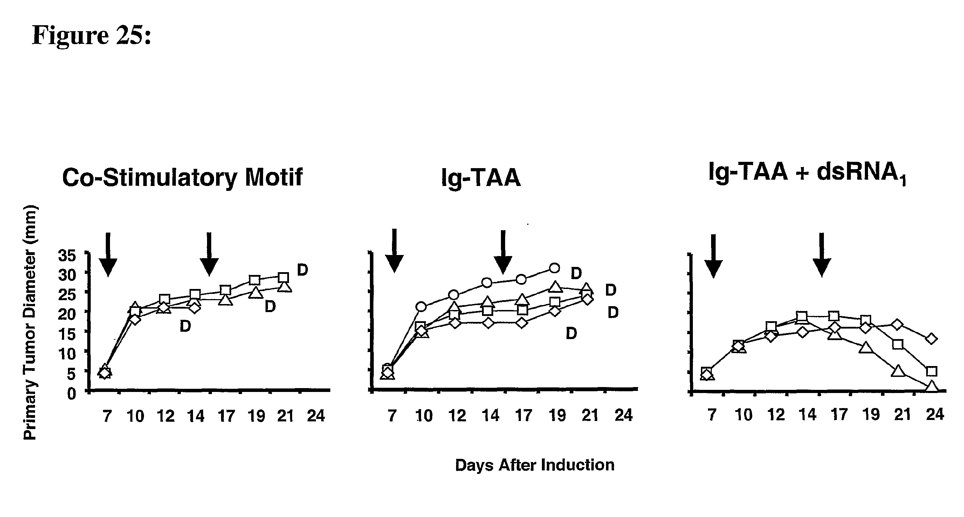

In conclusion, the results in Figure 25 show that both effective *in vivo* loading of APC with tumor associated antigen, together with simultaneous activation by selected synthetic RNA motifs are necessary and sufficient for effective control of tumor growth and induction of tumor rejection.

### Example 37. This Example, in context of sublethal inoculation of tumor cells, shows that the suboptimal response to tumor antigens could be corrected by therapy with peptide epitope within IgG backbone, together with co-stimulatory motif.

Balb/c mice were injected with SP2/0 cells stably expressing recombinant IgG (IgNP) that contains the MHC I (K^{d})epitope (amino acids 147-155) of WSN virus nucleoprotein in the CDR3 of the heavy chain. The cell inoculum was 1 million cells (in 100 µL) per mouse. The mice were observed until such time as palpable tumors were detected at the site of injection. At this point the tumors were measured and 8 mice were left untreated while 6 were injected intratumorally with purified IgTAA (i.e. purified IgNP, 2 mg/kg) and dsRNA (pApU, 4 mg/kg) weekly. Weekly measurements of the tumors were taken.

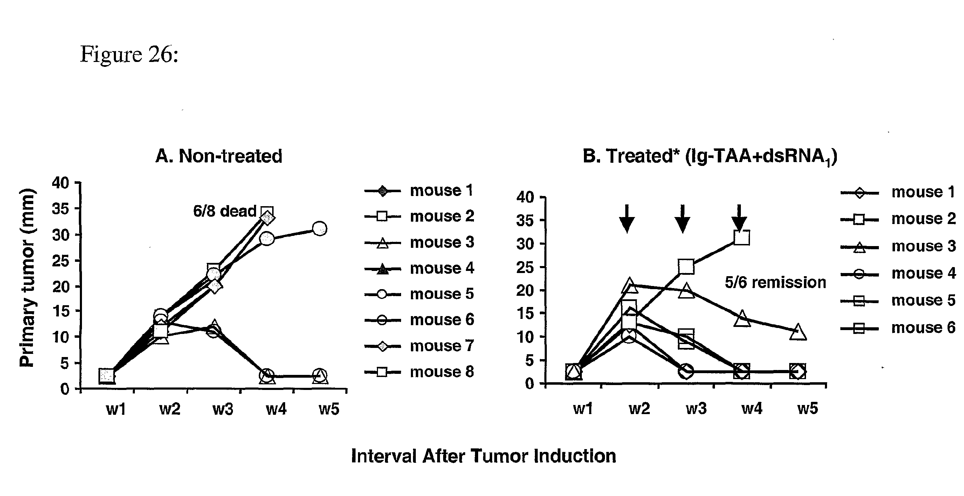

Panel A of Figure 26 shows that in 6 of 8 mice the induced tumor was progressive and ultimately lethal whereas 2 of the mice completely rejected the tumor spontaneously. Panel B of Figure 41 shows that the 3 weekly treatments with IgNP/dsRNA (indicated by the arrows) stimulated complete tumor rejection in 4 of the 6 mice and significant remission in another.

The results in Figure 26, panels A and B, show that both effective *in vivo* loading of APC with tumor associated antigen, together with simultaneous activation by selected synthetic RNA, can trigger an effective immune response to tumor-associated antigens.

### Example 38 shows that therapy of tumor-bearing mice with a tumor epitope within IgG backbone together with co-stimulatory synthetic RNA results in the restoration of the activatory status of tumor infiltrating lymphocytes.

Two BALB/c mice were injected with 10 million sp20 transfectoma expressing the NP-K^{d} epitope. After tumors developed, one mouse was injected intratumorally with 50 µg of selected dsRNA motif (pApU) plus 50µg of "IgNP" - recIgG-NP(K^{d}) in saline. The mice were sacrificed 24 hours later, tumors excised, digested with collagenase, filtered through 70um filter and viable cells isolated on Ficoll gradient. Cells were stained with mAbs against TCRβ, CD25 or isotype control and assessed by FACS analysis. The results were expressed as histograms, with percentage stained cells indicated.

### Materials:

SP20 cell line (ATCC);
2 BALB/c mice (Harland Sprague Dawley);
Falcon 70 micron filter(Becton Dickinson, cat# 352350);
Collagenase (Sigma, cat# C-9891);
BSA, fraction V (Sigma, cat# A-4503);
Collagenase buffer: 0.225gm BSA + 0.00625gm in 50 ml RPMI;
Ficoll-hypaque (1.077, Amersham, cat# 17-1440-02);
FACS Buffer:1% fetal calf serum + 0.1% azide in PBS;
Antibodies: All from BD Pharmingen; and,
Flow Cytometer: FACSCalibur (Becton Dickinson).

### Method: Tumor cell isolation and FACS analysis:

Tumor was induced as stated above 6 weeks prior;
Tumor was isolated from BALB/c mouse;
Tumor was minced with sterile scissors and 10ml of collagenase buffer added;
Incubate 40 minutes, 37°C;
Force tumor through a 70µm Falcon filter with a 3ml syringe plunger into a 50ml tube while washing with RPMI;
Wash 1X and resuspend in 4 mls warm RPMI buffer;
With equal volume of cell suspension layered over Ficoll, centrifuge at RT, 2000 RPM, for 15 minutes;
Isolate layer and wash once in HL-1 buffer and resuspend in FACS buffer to 2X10⁶/ml and run flow cytometry analysis;
Remaining cells were used for ELISPOT analysis;
Cells were placed in 12X75mm tubes, 50µl/tube and stained with FITC labeled anti-mouse antibody, 2µg/ tube plus 1µl/tube mouse serum:
   - Isotypic Control;
   - Anti -CD40;
   - Anti -CD8;
   - Anti -CD4;
   - Anti -CD25;
   - Anti -TCR gamma delta;
   - Anti -TCR Beta;
Incubate 30 minutes on ice; and,
Wash once with FACS buffer and resuspend in 300µl FACS buffer.

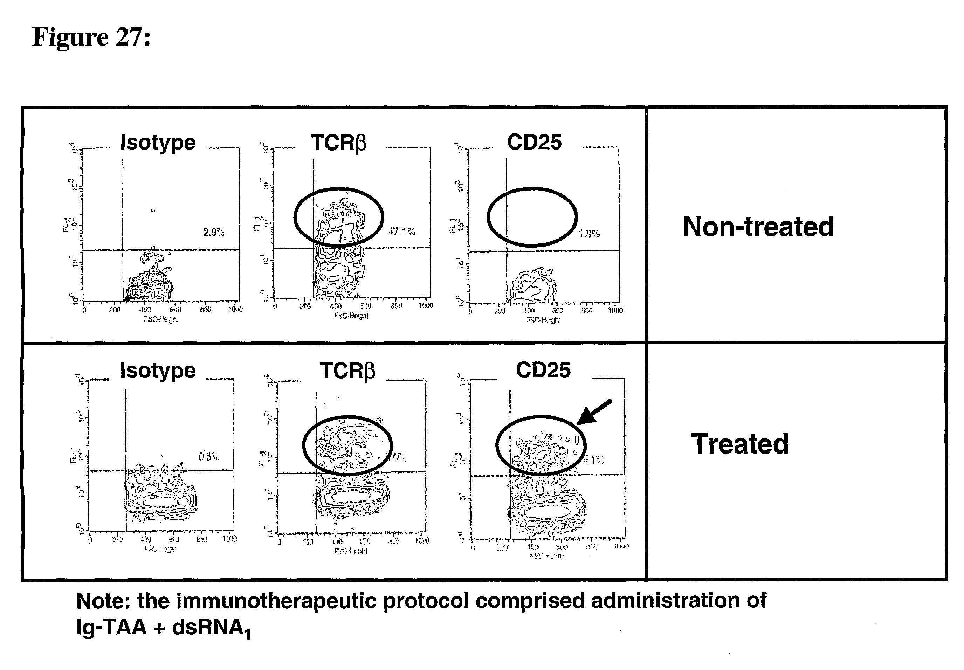

The results in Figure 27 show that tumor infiltrating lymphocytes displaying the T cell receptor marker TCRβ acquired expression of the activation marker CD25 upon treatment with recombinant immunoglobulin bearing tumor associated epitope, together with selected synthetic dsRNA motif.

Example 39 shows that successful therapy of tumor bearing mice with a peptide epitope within the IgG backbone together with a selected co-stimulatory molecule is associated with a specific differentiation pattern of Tc, comprising Tc1 in addition to Tc2.

Mice that rejected successfully the tumor following treatment with recombinant Ig carrying a tumor associated epitope together with selected synthetic dsRNA motif as explained in Example 37, were sacrificed and the T cell response against tumor associated epitope measured by ELISPOT analysis. The ELISPOT plates (Millipore, Molsheim, France) were incubated with purified anti-cytokine Abs (4ug/ml for anti-IL2 and anti-IL4, and 8 µg/ml for anti-IFN gamma, from BD Pharmingen) in sterile PBS (50 µl/well) at 4° C overnight. Next day, the plates were washed 2 times with DMEM media and blocked with 200µl/well of DMEM complete containing FBS, for an hour at 37° C. Single cell suspension was made from the spleens, red blood cells were lysed, cells washed, counted and incubated at 5x 10⁵ /well together with various concentrations of NP peptide. Plates were incubated 72 hours at 37 °C, 5% CO2. After 3 days, plates were washed 5 times with PBS-tween20 0.05% (washing buffer), and incubated with 100 µl /well of biotinylated anti-cytokine Abs, 2 µg /ml in PBS- tween20 0.05% - FBS 0.1%(ELISPOT buffer) overnight at 4 °C. Next day the plates were washed five times with washing buffer, and incubated for an hour with 1:1000 Streptavidin-HRP diluted in ELISPOT buffer. The reaction was developed with 3-amino-9-ethylcarbazole substrate (Sigma, St. Luis, MO) and stopped by washing the plate twice with tap water. Plates were then allowed to dry at room temperature for 24 hours.

The data were acquired using an automated system (Navitar, Rochester, NY) with ImagePro-Plus) software (Media Cybernetics, Silver Spring, MD). The results were expressed as number (mean ± SEM) of spot forming colonies corresponding to IL-4, IL-2 and IFN-γ. As a control, we used non-treated mice, which failed to reject tumor (n=4/group).

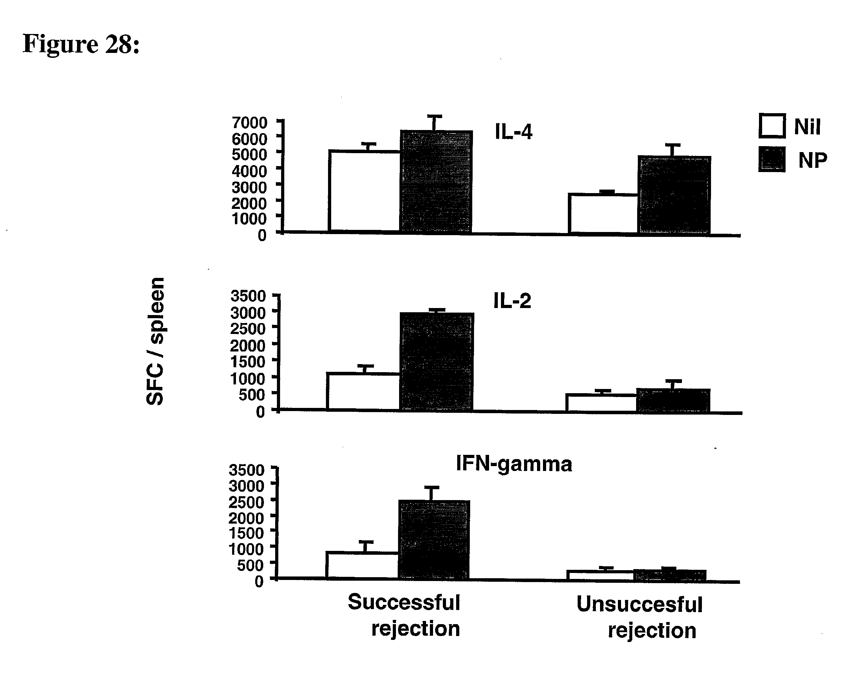

The results in Fig. 28 show that the treated mice that successfully rejected the tumor, developed Tc1 responses against the tumor associated epitope on the therapeutic Ig, along with Tc2 immunity. In contrast, the mice that failed to reject the tumor developed only Tc2 immunity.

### Example 40 shows induction of effective memory response subsequent to specific treatment of tumor bearing mice with a T cell epitope within IgG backbone, together with a selected co-stimulatory motif.

Mice bearing sp2/0 tumors expressing the NP-K^{d} TAA were treated as described in the Example 37, by injection with recombinant Ig bearing TAA together with selected synthetic RNA motifs. After tumor rejection, the mice were challenged by subcutaneous injection administered contralaterally, with 15 million SP2/0 cells expressing NP-Kd epitope. In parallel, 4 control naïve mice were similarly injected with a tumorigenic / lethal dose of same type of cells. The development and size of the tumors was monitored and represented as diameter (mm) versus time since challenge.

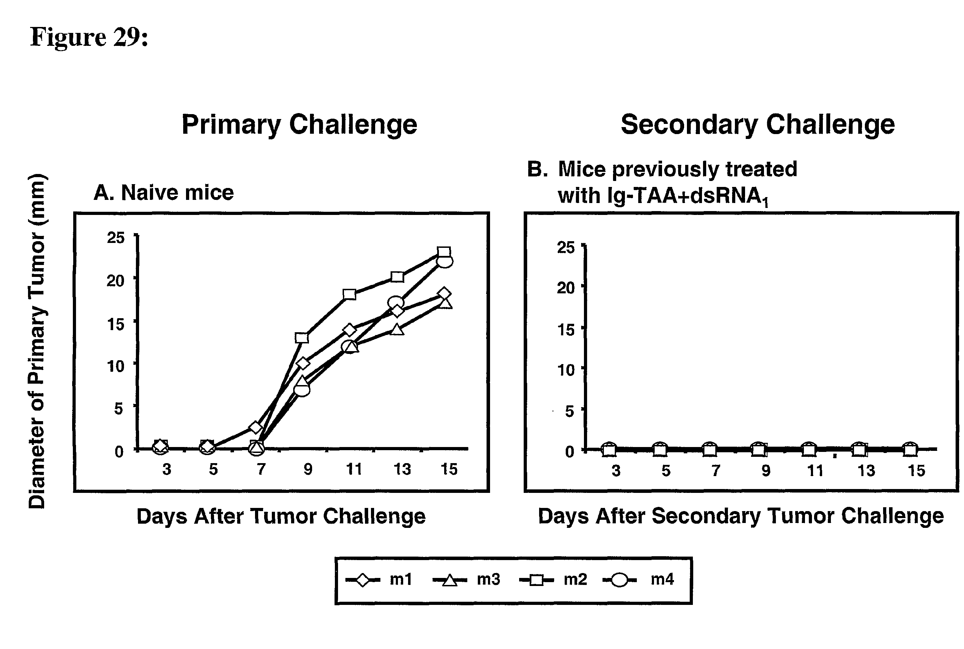

The results in Figure 29 show that successful rejection of tumor induced by indicated treatment is followed by effective protection against subsequent challenge with the same tumor, indicating development of effective immune memory.

### Example 41 shows that surprisingly, the induction of tumor rejection by an IgG bearing a TAA together with co-stimulator, results in cross-protection against a range of tumor cell variants lacking the TAA or displaying variants of TAA.

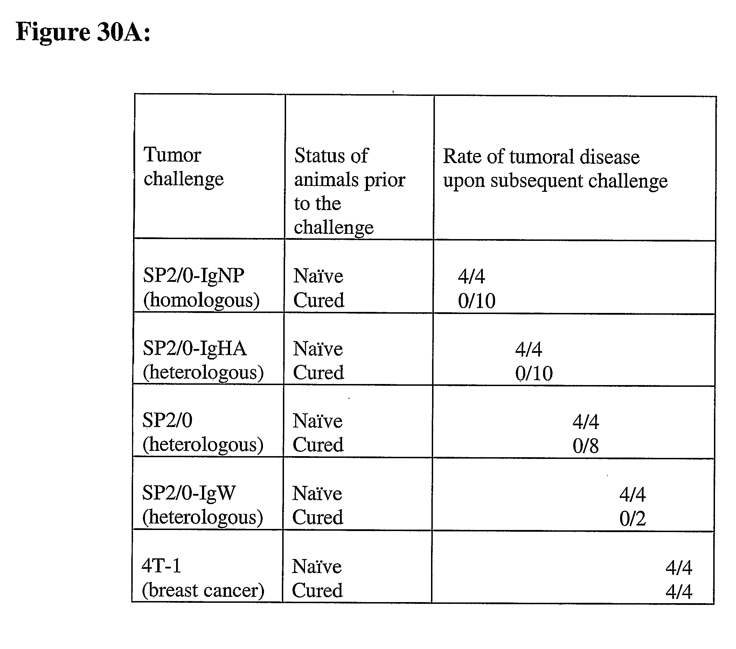

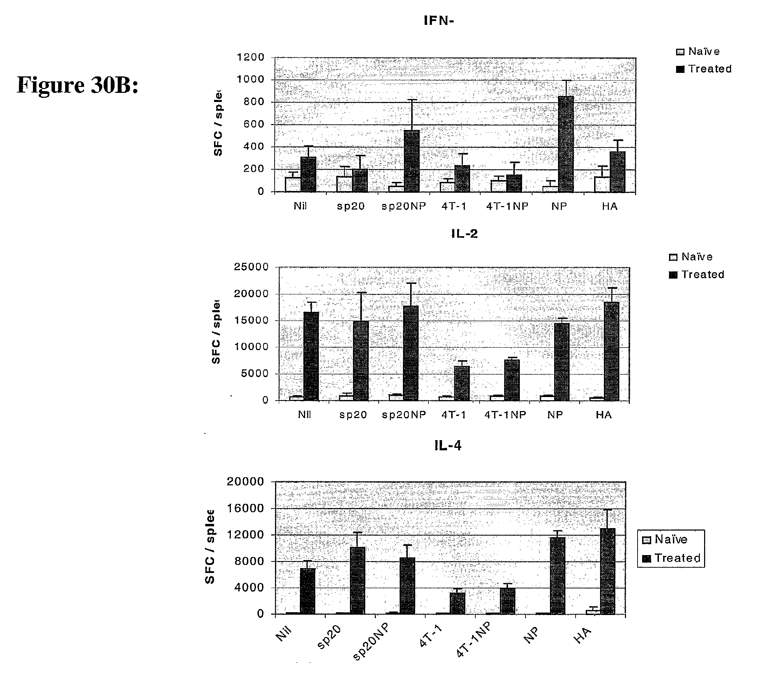

The mice protected against homologous challenge as described in Example 40, were subjected to sequential challenge with 15 million tumor cells representing the same tumor cells devoid of TAA (loss of antigen mutants) or bearing variants of TAA lacking the NP-K^{d} epitope. In addition, mice were challenged with a different type of tumor cell line (4T-1 adenocarcinoma) as a control, displayed in the table attached to Fig. 30A. In every case, naive controls were included.

The status of T cell immunity of mice protected against multiple challenges with tumor variants, has been assessed by ELISPOT analysis using splenic cell suspensions stimulated with TAA (NP-Kd peptide), HA (MHC class II-restricted peptide), or protein extracts from cell lysates. The ELISPOT plates (Millipore, Molsheim, France) were incubated with purified anti-cytokine Abs (4ug/ml for anti-IL2 and anti-IL4, and 8 µg/ml for anti-IFN gamma, from BD Pharmingen) in sterile PBS (50 µl/well) at 4°C overnight. Next day, the plates were washed 2 times with DMEM media and blocked with 200µl/well of DMEM complete containing FBS, for an hour at 37°C.

Single cell suspension was made from the spleens, red blood cells were lysed, cells washed, counted and incubated at 5x 10⁵ /well together with various concentrations of antigen. Plates were incubated 72 hours at 37 °C, 5% CO2. After 3 days, plates were washed 5 times with PBS-tween20 0.05% (washing buffer), and incubated with 100 µl /well of biotinylated anti-cytokine Abs, 2 µg /ml in PBS- tween20 0.05% - FBS 0.1%(ELISPOT buffer) overnight at 4 °C. Next day the plates were washed five times with washing buffer, and incubated for an hour with 1:1000 Streptavidin-HRP diluted in ELISPOT buffer. The reaction was developed with 3-amino-9-ethylcarbazole substrate (Sigma, St. Luis, MO) and stopped by washing the plate twice with tap water. Plates were then allowed to dry at room temperature for 24 hours.

The data were acquired using an automated system (Navitar, Rochester, NY) with ImagePro-Plus) software (Media Cybernetics, Silver Spring, MD). The results were expressed as number (mean ± SEM) of spot forming colonies corresponding to IL-4, IL-2 and IFN-γ. As a control, non-treated mice that failed to reject tumor (n=4/group) were used. As a control, naïve mice were included. The data are expressed as number (mean±SEM) of cytokine producing cells / organ (n=3/group).

The results in Figs. 30A - 30B (including the table in Fig. 30 A) show that the emerging immunity, subsequent to the indicated treatment that results in tumor rejection, protects against challenge with loss of antigen variants and is associated with overall expansion of cytokine producing cells. This indicates a broadening of the repertoire of anti-tumor lymphocytes, promoted by the proposed regimen, to tumor associated antigens that are not borne by the immunotherapeutic molecule.

The following numbered paragraphs (paras.) contain statements of broad combinations of the inventive technical features herein disclosed:-
1. A method of enhancing an immune response to an antigen comprising administering a composition comprised of double stranded RNA.
2. The method of paragraph 1 further comprising coadministering said composition with said antigen.
3. The method of paragraph 1 wherein the double stranded RNA consists of poly-denine and poly-uracil.
4. The method of paragraph 1 wherein the double stranded RNA consists of poly-guanine and poly-cytosine.
5. The method of paragraph 1 wherein the method enhances the Th1 and Tc1 cell response.
6. The method of paragraph 1 wherein the method enhances the B cell response to the antigen.
7. A method of preventing high-zone tolerance to a non-infectious antigen comprising administering said non-infectious antigen together with a double stranded RNA composition consisting of either poly-adenine and poly-uracil or poly-inosine and poly-cytosine.
8. The method of paragraph 7 wherein the method prevents B cell unresponsiveness.
9. The method of paragraph 7 wherein the antigen is a virus.
10. The method of paragraph 7 wherein the dsRNA is pA:pU.
11. A composition for enhancing an immune response to an antigen comprising a dsRNA sequence consisting of poly-adenine and poly-uracil.
12. The composition of paragraph 11 wherein said composition further comprises an antigen.
13. The composition of paragraph 11 wherein said antigen is administered in a pharmaceutically acceptable carrier.
14. The composition of paragraph 11 wherein said antigen is administered in an immunoglobulin.
15. The composition of paragraph 11 wherein said pharmaceutically acceptable is IgG.
16. The composition of paragraph 11 wherein the antigen is a tumor associated epitope.
17. The composition of paragraph 12 wherein the antigen is a virus.
18. The composition of paragraph 11 wherein the antigen is a tumor associated T cell epitope.
19. A composition for enhancing an immune response to an antigen comprising a dsRNA sequence consisting of poly-inosine and poly-cytosine.
20. The composition of paragraph 19 wherein said composition further comprises an antigen.

## Claims

1. Double stranded poly-adenine:poly-uracil RNA (pA:pU) for use in a method of enhancing an immune response to an antigen, the method comprising administering pA:pU to a patient, wherein the method induces a Tc1 cell response in the patient.

2. pA:pU for use in a method of enhancing an immune response to an antigen according to claim 1 wherein the method induces MHC class 1-restricted T cells, producing IFN-γ.

3. pA:pU for use in a method of enhancing an immune response to an antigen according to claim 1 or claim 2 wherein the pA:pU is coadministered with the antigen.

4. pA:pU for use in a method of enhancing an immune response to an antigen according to any one of the preceding claims wherein pA:pU is administered mucosally or systemically.

5. pA:pU for use in a method of enhancing an immune response to an antigen according to any one of the preceding claims wherein the pA:pU has a molecular weight of greater than 50kDa.

6. pA:pU for use according to any one of the preceding claims wherein the antigen is a protein antigen.

7. pA:pU for use in a method of enhancing an immune response to an antigen according to any one of the preceding claims wherein the antigen is a non-infectious antigen.

8. pA:pU for use in a method of enhancing an immune response to an antigen according to any one of the preceding claims wherein the method of enhancing an immune response forms part of a treatment of cancer.

9. pA:pU for use in a method of enhancing an immune response to an antigen according to any one of the preceding claims wherein the antigen is a tumor associated epitope.

10. pA:pU for use in a method of enhancing an immune response to an antigen according to claim 9 wherein the antigen is a tumor associated T cell epitope.

11. pA:pU for use in a method of enhancing an immune response to an antigen according to any one of claims 1 to 7 wherein the method of enhancing an immune response forms part of a method of virus immunization or treatment of viral infection.

12. pA:pU for use in a method of enhancing an immune response to an antigen according to claim 11 wherein the antigen is a virus.

13. pA:pU for use according to any one of the preceding claims wherein the patient is a human.

14. Use of double stranded poly-adenine:poly-uracil RNA (pA:pU) in the manufacture of a medicament for use in a method of enhancing an immune response to an antigen in a patient, wherein the method induces a Tc1 cell response in the patient.

15. Use according to claim 14 wherein the method involves coadministration of the pA:pU with the antigen.
